# EUROPEAN PATENT APPLICATION

(11) **EP 1 205 490 A1**
(43) Date of publication of application: **15.05.2002**
(21) Application number: 00124629.7
(22) Date of filing: 10.11.2000
(51) Int. Cl.: C07K 19/00, C07K 14/005, C12N 5/10, C12N 15/33

(54) **Fusion protein comprising integrase (phiC31) and a signal peptide (NLS)**

(71) Applicant: ARTEMIS Pharmaceuticals GmbH, 51063 Köln (DE)
(72) Inventor: Kühn, Ralf, Dr., 50937 Köln (DE); Felder, Susanne, Dr., 41469 Neuss (DE); Schwenk, Frieder, Dr., 50999 Köln (DE); Küter-Luks, Birgit, 50668 Köln (DE); Faust, Nicole, 51063 Rösrath (DE)
(74) Representative: Helbing, Jörg, Dr. Dipl.-Chem.

(57) **Abstract**

The present invention concerns a fusion protein consisting of the site-specific DNA recombinase C31-Int of phage ΦC31 and a peptide sequence which directs the nuclear uptake of the fusion protein in eucaryotic cells, and the use of this fusion protein to recombine DNA molecules containing C31-Int recognition sequences in eucaryotic cells at high efficiency. In addition the invention relates to a cell, preferably a mammalian cell which contains said C31-Int recognition sequences in its genome and which are recombined by the said C31-Int fusion protein. Moreover, the invention relates to the use of said cell to study the function of genes and for the creation of transgenic organisms to study gene function at various developmental stages, including the adult. In conclusion, the present invention provides a process which enables the highly efficient modification of the genome of mammalian cells by site-specific recombination.

## Description

The present invention concerns a fusion protein consisting of the site-specific DNA recombinase C31-Int of phage ΦC31 and a peptide sequence which directs the nuclear uptake of the fusion protein in eucaryotic cells, and the use of this fusion protein to recombine DNA molecules containing C31-Int recognition sequences in eucaryotic cells at high efficiency. In addition the invention relates to a cell, preferably a mammalian cell which contains said C31-Int recognition sequences in its genome and which are recombined by the said C31-Int fusion protein. Moreover, the invention relates to the use of said cell to study the function of genes and for the creation of transgenic organisms to study gene function at various developmental stages, including the adult. In conclusion, the present invention provides a process which enables the highly efficient modification of the genome of mammalian cells by site-specific recombination.

### Background of the invention

The controlled and permanent modification of the genome of eucaryotic cells and organisms is an important method for research applications, e.g. for studying gene function, for medical applications like gene therapy and the creation of disease models and for the design of economically important animals and crops. The basic methods for genome manipulations by the engineering of endogenous genes through gene targeting in murine embryonic stem (ES) cells are well established and used since many years (Capecchi, Trends in Genetics, 5, 70-76 (1989)). Since ES cells can pass mutations induced in vitro to transgenic offspring in vivo it is possible to analyse the consequences of gene disruption in the context of the entire organism. Thus, numerous mouse strains with functionally inactivated genes ("knock-out mice") have been created by this technology and utilised to study the biological function of a variety of genes (Koller et al., Ann. Rev. Immunol., 10, 705 - 730 (1992)). More importantly, mouse mutants created by this procedure (also known as "conventional, complete or classical mutants"), contain the inactivated gene in all cells and tissues throughout life. Thus, classical mouse mutants represent the best animal model for inherited human diseases as the mutation is introduced into the germline but are not the optimal model to study gene function in adults, e.g. to validate potential drug target genes. A refined method of targeted mutagenesis, referred to as conditional mutagenesis, employs the Cre/loxP site-specific recombination system which enables the temporally and/or spatially restricted inactivation of target genes in cells or mice (Rajewsky et al., J. Clin. Invest., 98, 600-603 (1996)). The phage P1 derived Cre recombinase recognises a 34 bp sequence referred to as loxP site which is structured as an inverted repeat of 13 bp separated by an assymmetric 8 bp sequence which defines the direction of the loxP site. If two loxP sites are located on a DNA molecule in the same orientation the intervening DNA sequence is excised by Cre recombinase from the parental molecule as a closed circle leaving one loxP site on each of the reaction products (Kilby et al., TIG, 9, 413-421 (1993)). The creation of conditional mouse mutants initially requires the generation of two mouse strains, one containing two or more Cre recombinase recognition (loxP) sites in its genome while the other harbours a Cre transgene. The former strain is generated by homologous recombination in ES cells as described above, except that the exon(s) of the target gene is (are) flanked by two loxP sites which reside in introns and do not interfere with gene expression. The Cre transgenic strain contains a transgene whose expression is either constitutively active in certain cells and tissues or is inducible by external agents, depending on the promoter region used. Crossing of the loxP-flanked mouse strain with the Cre recombinase expressing strain enables the deletion of the loxP-flanked exons in the genome of doubly transgenic offspring in a prespecified temporally and/or spatially restricted manner. Thus, the method allows the analysis of gene function in particular cell types and tissues of otherwise widely expressed genes. Moreover, it enables the analysis of gene function in the adult organism by circumventing embryonic lethality which is often the consequence of complete (germline) gene inactivation. For pharmaceutical research, aiming to validate the utility of genes and their products for drug development, gene inactivation which is inducible in adults provides an excellent genetic tool as this mimicks the biological effects of target inhibition upon drug application.

Since the first description of the concept of conditional gene targeting using the Cre/loxP system in mice in 1994 (Gu et al., Science 265, 103-106 (1994)) this method became increasingly popular among the research community and resulted in a broad collection of genetic tools for biological research in the mouse. More than 30 Cre transgenic mouse strains with various tissue specificities for gene inactivation have been created, including several "deleter" strains which allow to remove the loxP-flanked target gene segment in the male or female germline (Cohen-Tannoudji et al., Mol. Hum. Reprod. 4, 929-938 (1998); Metzger et al., Curr. Op. Biotech., 10, 470-476 (1999)). The need to characterise the expression pattern of Cre mediated recombination in newly generated strains stimulated the construction of a number of "Cre-reporter" strains which harbour a silent reporter gene the expression of which is activated upon Cre-mediated deletion (Nagy, Genesis, 26, 99-109 (2000)). Conditional mouse mutants have been reported for about 20 different genes, many of them could not be studied in adults as their complete inactivation leads to embryonic lethality (Cohen-Tannoudji et al., Mol. Hum. Reprod. 4, 929-938 (1998)).

Great efforts have also been made to control the expression of Cre recombinase in an inducible fashion in mice. After the first demonstration that inducible gene knock-out is feasible in adult mice using an interferon controlled promoter (Kühn et al., Science, 269, 1427-1429 (1995)), mainly two methods were applied to control the activity of Cre recombinase. First, it has been demonstrated that the fusion of Cre with the ligand binding domain of a mutant estrogen receptor allows to control recombinase activity by a specific steroid-like inducer. Several transgenic mouse strains expressing such a fusion protein have been generated and allow to induce gene inactivation in specific tissues (Metzger et al., Curr. Op. Biotech., 10, 470-476 (1999)). Furthermore, the tetracycline-regulated gene expression system has been successfully used to control the expression of Cre in transgenic mice and thus provides a second system for inducible gene inactivation using doxycycline as inducer (Saam et al., J. Biol. Chem. 274, 38071-38082 (1999)).

In addition to the application of Cre/loxP for gene inactivation by deletion of a gene segment this recombination system has been proved to be useful also for a number of other genomic manipulations in ES cells or mice. These include the conditional activation of transgenes in mice, chromosome engineering to obtain deletion, translocation or inversion, the simple removal of selection marker genes, gene replacement, the targeted insertion of transgenes and the (in)activation of genes by inversion (Nagy, Genesis, 26, 99-109 (2000); Cohen-Tannoudji et al., Mol. Hum. Reprod. 4, 929-938 (1998)). In conclusion, the Cre/loxP recombination system has been proven to be extremely useful for the analysis of gene function in mice by broadening the methodological spectrum for genome engineering. It can be expected that many of the protocols now established for the mouse may be applied in future also to other animals or plants.

In contrast to the huge diversity of genome manipulations which have been developed for the Cre/loxP system, very limited efforts have been made to develop further site-specific recombination systems for the use in mammalian cells. Alternative recombination systems of different specificity but with an efficiency comparable to Cre/loxP could further enhance the flexibility of genome engineering by the side to side use of two or more systems in the same cell or organism. Furthermore, unidirectional recombination systems which follow a different mechanism than the reversible Cre/loxP-mediated recombination should allow to develop new applications for genome engineering which cannot be performed with the current systems.

The reasons for the almost exclusive use of the Cre/loxP system for site-specific recombination in mammalian cells are readily explained by a number of requirements which must be fulfilled for the efficient use of a recombinase in mammalian cells:
i) the recombinase should act independent of cofactors like helper proteins,
ii) it should act independent of the supercoiling status of the target DNA and also on mammalian chromatin,
iii) it should be efficiently active and stable at a temperature of 37°C, and
iv) it should recognize a target sequence which is sufficiently long to be unique among large genomes, and it should exhibit a very high affinity to its target site for efficient action (Kilby et al., TIG, 9, 413-421 (1993)).

Among the more than 200 described members of the integrase and resolvase/invertase recombinase families only the Cre/loxP system is presently known to fulfill all of these requirements(Nunes-Düby et al., Nucleic Acids Res., 26, 391-406 (1998); Kilby et al., TIG, 9, 413-421 (1993); (Ringrose et al., J. Mol. Biol., 284, 363 - 384 (1998)). Besides Cre/loxP a few recombinases have been shown to exhibit some activity in mammalian cells but their practical value is presently unclear as their efficieny has not been compared to the Cre/loxP system on the same genomic recombination substrate and in some cases it is known that one or more of the criteria listed above are not met. The best characterised examples are the yeast derived FLP and Kw recombinases which exhibit a temperature optimum at 30°C but which are unstable at 37°C (Buchholz et al., Nature Biotech., 16, 657 - 662 (1998); Ringrose et al., Eur. J. Biochem., 248, 903- 912). For FLP it has been shown in addition that its affinity to the FRT target site is much lower as compared to the affinity of Cre to loxP sites (Ringrose et al., J. Mol. Biol., 284, 363 - 384 (1998)). Other recombinases which show in principle some activity in mammalian cells are a mutant integrase of phage λ, the integrases of phages ΦC31 and HK022, mutant γδ-resolvase and β-recombinase (Lorbach et al., J. Mol. Biol., 296, 1175 - 81 (2000); Groth et al., Proc. Natl. Acad. Sci. USA, 97, 5995 - 6000 (2000); Kolot et al., Mol. Biol. Rep. 26, 207 - 213 (1999); Schwikardi et al., FEBS Lett., 471, 147 - 150 (2000); Diaz et al., J. Biol. Chem., 274, 6634 - 6640 (1999)). However, the practical value of these recombinases for use in mammalian cells is limited as their efficiency to recombine mammalian genomic DNA has not been compared with the Cre/loxP system. However, from the available data it can be assumedthat these recombinases are much less effective than the Cre/loxP system.

In a few cases attempts have been made to improve the performance of recombinases in mammalian cells: for FLP a mutant showing improved thermostability and acticity at 37°C has been isolated but this mutant is still considerably more heat labile as compared to Cre (Buchholz et al., Nature Biotech., 16, 657 - 662 (1998)). In the case of λ-integrase and γδ-resolvase the absolute requirement for coproteins and supercoiled DNA could be eliminated by the introduction of specific point mutations.

The import of cytoplasmic proteins into the nucleus of eucaryotic cells through nuclear pores is a regulated, energy dependent process mediated by specific receptors (Görlich et al., Science, 271, 1513 - 1518 (1996)). Proteins which do not possess a signal sequence recognised by the nuclear import machinery are excluded from the nucleus and remain in the cytoplasm. Numerous of such nuclear localisation signal sequences (NLS), which share a high proportion of basic amino acids in common, have been characterised (Boulikas, Crit. Rev. Eucar. Gene Expression, 3, 193 - 227 (1993)), the prototype of which is the 7 amino acid NLS derived from the T-antigen of the SV40 virus (Kalderon et. al, Cell, 39, 499 - 509 (1984)).

However, for Cre recombinase it has been shown that the addition of this nuclear localisation sequence does not improve but slightly decreases its recombination efficiency in mammalian cells (Le et al., Nucleic Acids Res., 27, 4703 - 4709 (1999)).

The level of activity exhibited by recombinases of diverse prokaryotic origin in mammalian cells may be the result of the intrinsic properties of an enzyme depending on parameters like its temperature optimum, its target site affinity, protein structure and stability, the degree of cooperativity, the stability of the synaptic complex and the dependence on coproteins or supercoiled DNA. Within the specific environment of mammalian cells the activity of a prokaryotic recombinase could be limited by additional factors such as a short halflife of the recombinase transcript, a short halflife of its protein, its inability to act on histone-complexed and higher order structured mammalian genomic DNA, exclusion from the nucleus or the recognition of cryptic splice sites within its mRNA resulting in a nonfunctional transcript. Due to the lack of information on the parameters listed above for almost all recombinases it is presently not possible to rationally optimise their performance in mammalian cells.

### Summary of the Invention

The object to be solved by the invention of the present application is the provision of an alternative recombination system to the Cre/loxP system which has a different specificity but an efficiency comparable to Cre/loxP. It was found that the above object can be solved by a modified form of the Integrase of phage ΦC31 (hereinafter C31-Int) which, in contrast to its wildtype form, allows the highly efficient recombination of extrachromosomal and chromosomal DNA in mammalian cells. In said modified C31 Int C31-Int is fused with a peptide directing nuclear protein import.

The present invention thus provides:
(1) a fusion protein comprising
   (a) an integrase domain being a phage ΦC31 integrase (C31-Int) protein or a mutant thereof, and
   (b) a signal peptide domain being linked to (a) and directing the nuclear import of said fusion protein into eucaryotic cells;
(2) a DNA coding for the fusion protein as defined in (1) above;
(3) a vector containing the DNA as defined in (2) above;
(4) a microorganism containing the DNA of (2) above and/or the vector of (3) above;
(5) a process for preparing the fusion protein as defined in (1) above which comprises culturing a microorganism as defined in (4) above;
(6) the use of the fusion protein as defined in (1) above to recombine DNA molecules containing C31-Int recognition sequences in eucaryotic cells;
(7) a cell, preferably a mammalian cell containing the DNA sequence of (2) above in its genome;
(8) the use of the cell of (7) above for studying the function of genes and for the creation of transgenic organisms;
(9) a transgenic organism, preferably a transgenic mammal containing the DNA sequence of (2) above in its genome;
(10) the use of the transgenic organism of (9) above for studying gene function at various developmental stages; and
(11) a method for recombining DNA molecules of cells or organisms containing C31-Int recognition sequences which comprises supplying the cells or organisms with a fusion protein as defined in (1) above, or with a DNA sequence of (2) above and/or a vector of (3) above which are capable of expressing said fusion protein in the cell or organism.

The present invention combines the use of the prokaryotic C31-Int recombinase with a eukaryotic signal sequence which increases its efficiency in mammalian cells such that it equal to the widely used Cre/loxP recombination system. Thus, the improved C31-Int recombination system provides an alternative recombination system for use in mammalian cells and organisms which allows to perform the same types of genomic modifications as shown for Cre/loxP, including conditional gene inactivation by recombinase-mediated deletion, the conditional activation of transgenes in mice, chromosome engineering to obtain deletion, translocation or inversion, the simple removal of selection marker genes, gene replacement, the targeted insertion of transgenes and the (in)activation of genes by inversion.

### Short Description of Figures

Fig. 1: C31-Int and Cre recombinase expression vectors and a recombinase reporter vector used for transient and stable transfections

Fig. 2: Results of transient transfections of C31-Int and Cre recombinase and reporter vectors into CHO cells.

Fig. 3: Results of transient transfections of C31-Int and Cre recombinase vectors into a stable reporter cell line.

Fig. 4: In situ detection of β-galactosidase in 3T3(pRK64)-3 cells transfected with recombinase expression vectors

Fig. 5: Test vector for C31-Int mediated deletion, pRK64, and the expected deletion product.

Fig. 6: PCR products generated with the primers P64-1 and P64-4 and sequence comparison.

### Detailed Description of the Invention

The "organisms" according to the present invention are multi-cell organisms and can be vertebrates such as mammals (humans and non-human animals including rodents such as mice or rats) or non-mammals (e.g., fish), or can be invertebrates such as insects or worms, or can be plants (higher plants, algi or fungi). Most preferred living organisms are mice and fish.

"Cells" and "eucaryotic cells" according to the present invention include cells isolated from the above defined living organism and cultured *in vitro.* These cells can be transformed (immortalized) or untransformed (directly derived from the living organism; primary cell culture).

"Microorganism" according to the present invention relates to procaryotes (e.g. E.coli) and eucaryotic microorganisms (e.g. yeasts).

In embodiment (1) of the present invention, i.e. the fusion protein, the integrase domain is preferably a C31-Int having the amino acid sequence shown in SEQ ID NO:21 or a C-terminal truncated form thereof. Suitable truncated forms of the C31-Int comprise aa 306 to 613 of SEQ ID NO:21.

The signal peptide domain (hereinafter also referred to as "NLS") is preferably derived from yeast GAL4, SKI3, L29 or histone H2B proteins, polyoma virus large T protein, VP1 or VP2 capsid protein, SV40 VP1 or VP2 capsid protein, Adenovirus E1a or DBP protein, influenza virus NS1 protein, hepatitis virus core antigen or the mammalian lamin, c-myc, max, c-myb, p53, c-erbA, jun, Tax, steroid receptor or Mx proteins (see Boulikas, Crit. Rev. Eucar. Gene Expression, 3, 193 - 227 (1993)), simian virus 40("SV40") T antigen (Kalderon et. al, Cell, 39, 499 - 509 (1984)) or other proteins with known nuclear localisation. The NLS is preferably derived from the SV40 T-antigen.

Furthermore, the signal peptide domain preferably has a length of 5 to 74, preferably 7 to 15 amino acid residues. More preferred is that the signal peptide domain comprises a segment of 6 amino acid residues wherein at least 2 amino acid residues, preferably at least 3 amino acid residues are positively charged basic amino acids. Basic amino acids include, but are not limited to, Lysin, Arginin and Histidine. Particularly preferred signal peptides are show in the following table.

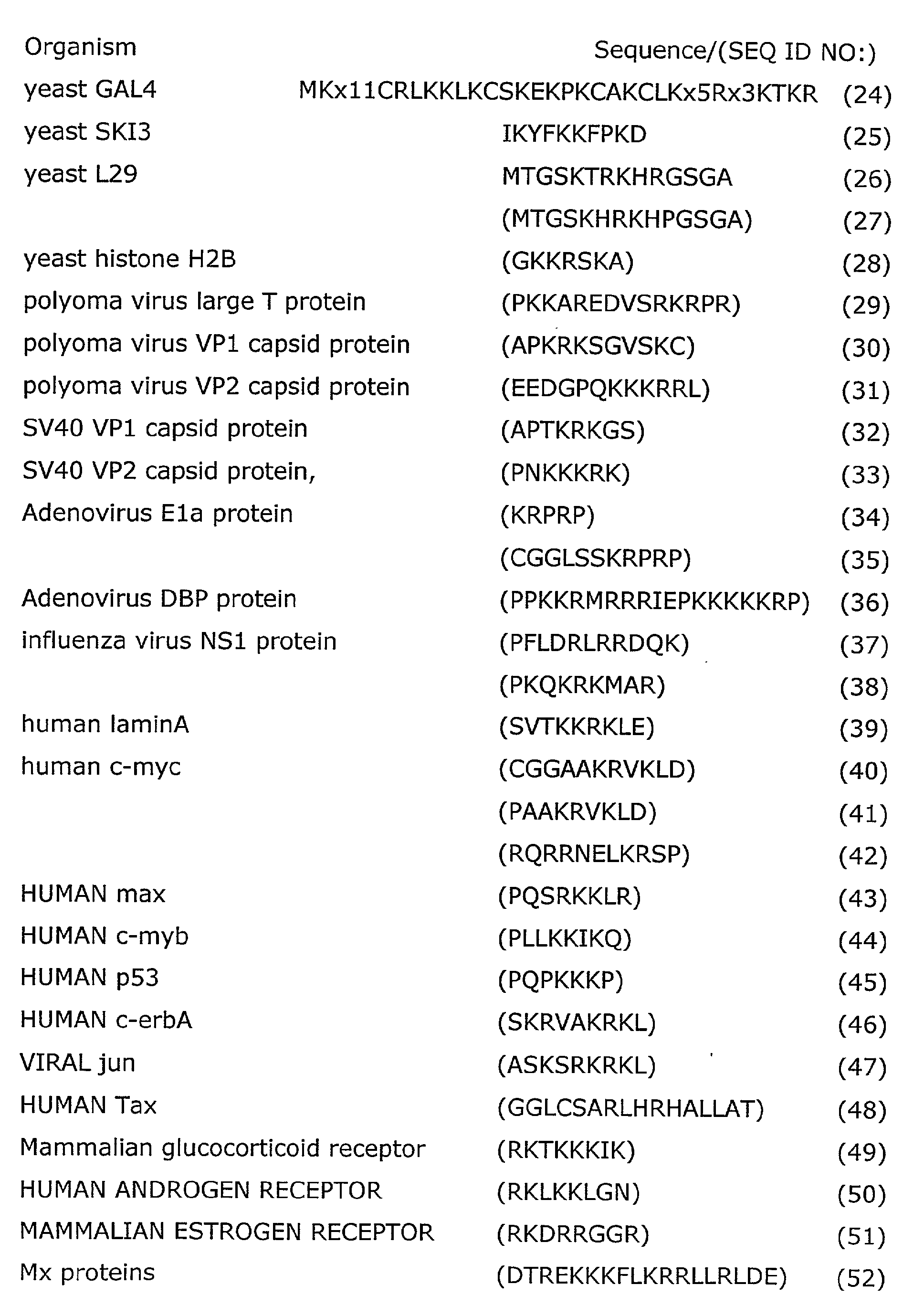

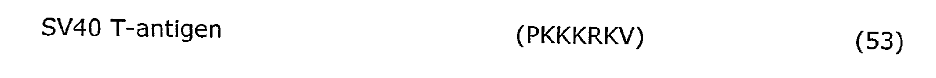

The most preferred signal peptide domain is that of SV40 T-antigen having the sequence Pro-Lys-Lys-Lys-Arg-Lys-Val.

The signal peptide domain may be linked to the N-terminal or C-terminal of the integrase domain or may be integrated into the integrase domain, preferably the signal peptide domain is linked to the C-terminal of the integrase domain. According to the present invention, the signal peptide domain may be linked to the integrase domain directly or through a linker peptide. Suitable linkers include peptides having from 1 to 30, preferably 1 to 15 amino acid residues, said amino acid residues being essentially neutral amino acids such as Gly, Ala and Val.

The most preferred fusion protein of the present invention comprises the amino acid sequence shown in SEQ ID NO:23.

In embodiments (7) and (9) of the invention the cell or transgenic mammal also contains C31-Int recognition sequences in its genome. The term "mammal" as used in embodiment (9) of the invention includes non-human mammals (viz. animals as defined above) and humans (if such subject matter is patentable with the respective patent authority).

Since the modified C31-Int recombinase acts in mammalian cells as efficient as the widely used Cre/loxP system it can be used for a large variety of genomic modifications. Concerning embodiment (10) it is to be noted that the mammals of embodiment (9) can be used to study the function of genes, e.g., in mice, by conditional gene targeting. For this purpose one attP and one attB site in the same orientation can be introduced into introns of a gene by homologous recombination of a gene targeting vector in ES cells such that the two sites flank one or more exons of the gene to be studied but do not interfere with gene expression. A selection marker gene, needed to isolate recombinant ES cell clones, can be flanked by two recognition sites of another recombinase such as loxP or FRT sites to enable deletion of the marker gene upon transient expression of the respective recombinase in ES cells. These ES cells can be used to generate germline chimaeric mice which transmit the target gene modified by att sites to their offspring and allow to establish a modified mouse strain. The crossing of this strain with a C31-Int recombinase transgenic line or the application of C31-Int protein will result in the deletion of the att-flanked gene segment from the genome of doubly transgenic offspring and the inactivation of the target gene in doubly transgenic offspring in a prespecified temporally and/or spatially restricted manner. The C31-Int transgenic strain contains a transgene whose expression is either constitutively active in certain cells and tissues or is inducible by external agents, depending on the promoter region used. If an attB and an attP site are placed into the genome in opposite orientation C31-Int mediated recombination results in the irreversible inversion of the flanked gene segment leading the functional loss of on or more exons of the target gene. Thus, the method allows the analysis of gene function in particular cell types and tissues of otherwise widely expressed genes and circumvents embryonic lethality which is often the consequence of complete (germline) gene inactivation. For the validation of genes and their products for drug development, gene inactivation which is inducible in adults provides an excellent genetic tool as this mimicks the biological effects of target inhibition upon drug application. If a pair of attB/P sites is placed in the same or opposite orientation into a chromosome at large distance using two gene targeting vectors, C31-Int recombination allows to delete or invert chromosome segments containing one or more genes, or chromosomal translocations if the two sites are located on different chromosomes. In another application of the method a pair of attB/P sites is placed in the same orientation within a transgene such that the deletion of the att-flanked DNA segment results in gene expression, e.g. of a toxin or reporter gene for cell lineage studies, or in the inactivation of the transgene.

In addition, according with embodiment (11) of the invention the C31-Int recombination system can be also used for the site specific integration of foreign DNA into the genome of mammalian cells, e.g. for gene therapy. For this purpose only one attB (or attP) site is initially introduced into the genome by homologous recombination or an endogenous genomic sequence which resembles attB or attP is used . The application of a vector containing an attP (or attB) site to such cells or mice in conjunction with the expression of C31-Int recombinase will lead to the site specific integration of the vector into the genomic att site.

Thus, the present invention provides a process which enables the highly efficient modification of the genome of mammalian cells by site-specific recombination. Said process possesses the following advantages over current technology:
(i) the modified C31-Integrase allows to recombine extrachromosomal and genomic DNA in mammalian cells at much higher efficiency as compared to the use of its wildtpe form;
(ii) the modified C31-Integrase is the first described alternative recombination system with equal efficiency to Cre/loxP for the recombination of chromosomal DNA in mammalian cells.

The appended figures further explain the present invention:

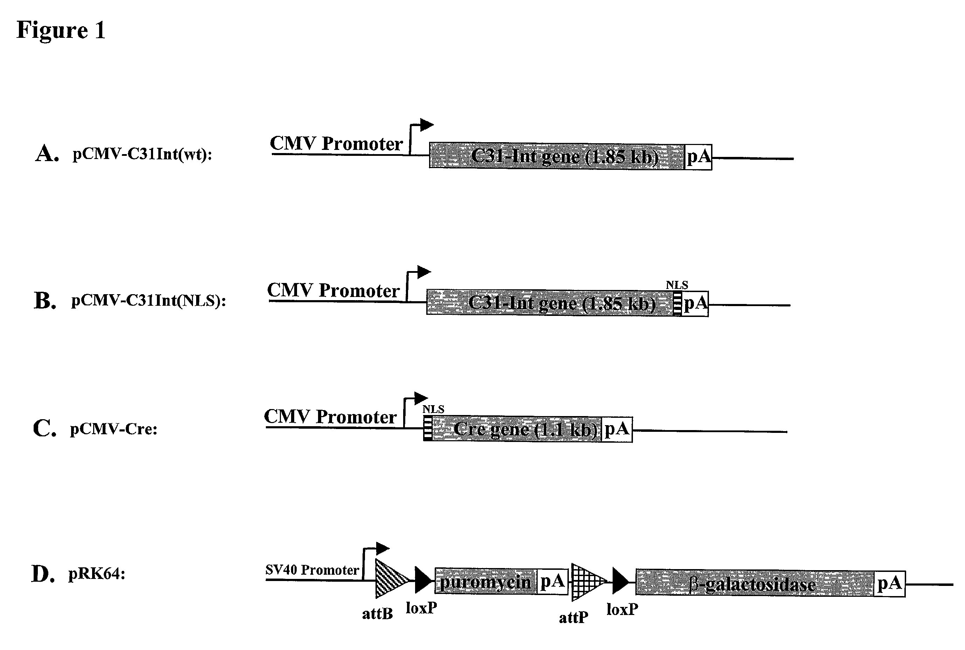

Figure 1 shows C31-Int and Cre recombinase expression vectors and a recombinase reporter vector used for transient and stable transfections A-C: Mammalian expression vectors for recombinases which contain the CMV immediate early promoter followed by a hybrid intron, the coding region of the recombinase to be tested, and an artificial polyadenylation signal sequence (pA).
   A: pCMV-C31Int(wt) containing the nonmodified (wildtype) 1.85 kb coding region of C31-Int as found in the genome of phage ΦX31.
   B: pCMV-C31Int(NLS) containing a modified C31-Int gene coding for the full length C31-Int protein with a C-terminal fusion to the SV40 virus large T antigen nuclear localisation signal (NLS).
   C: pCMV-Cre contains the 1.1 kb Cre coding region with an N-terminal fusion of the SV40 T antigen NLS.
   D: Recombination substrate vector pRK64 contains a SV40 promoter region followed by a 1.1 kb cassette consisting of the coding region of the puromycin resistance gene and a polyadenylation signal sequence, flanked 5' by the 84 bp attB and 3' by the 84 bp attP recognition site of C31-Int. pRK64 contains in addition two Cre recognition (loxP) sites in direct orientation next to the att sites.

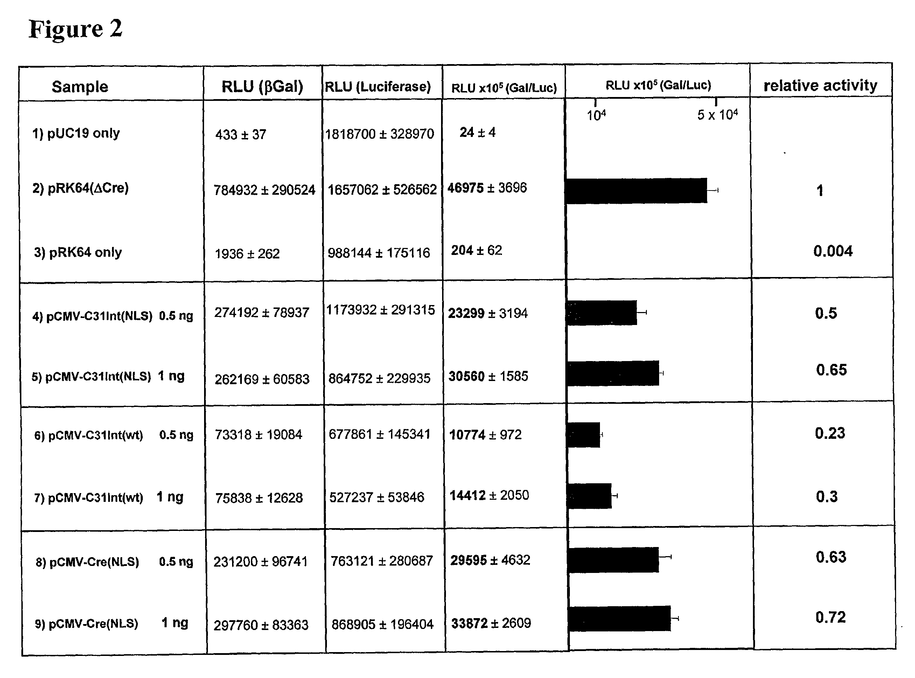

Figure 2 shows results of transient transfections of C31-Int and Cre recombinase and reporter vectors into CHO cells.
   All transfections were performed with a fixed amount of the reporter plasmid pRK64 and 0.5 ng or 1 ng of the recombinase expression plasmids pCMV-C31-Int(NLS) (samples 4-5), pCMV-C31-Int(wt) (samples 6-7) or pCMV-Cre (samples 8-9). Negative controls: transfection with pRK64 (sample 3) or pUC19 alone (sample 1). Positive control: transfection with the Cre-recombined reporter pRK64(ΔCre) (sample 2).
   The vertical rows show the mean values and standard deviation of relative light units obtained from lysates with the assay for β-galactosidase (RLU(β-Gal), the RLU from the assay for Luciferase, the ratio of the β-galactosidase and Luciferase values with standard deviation (RLU x 10⁵ (Gal/Luc)), and the relative activity of the various recombinases as compared to the positive control defined as 1.

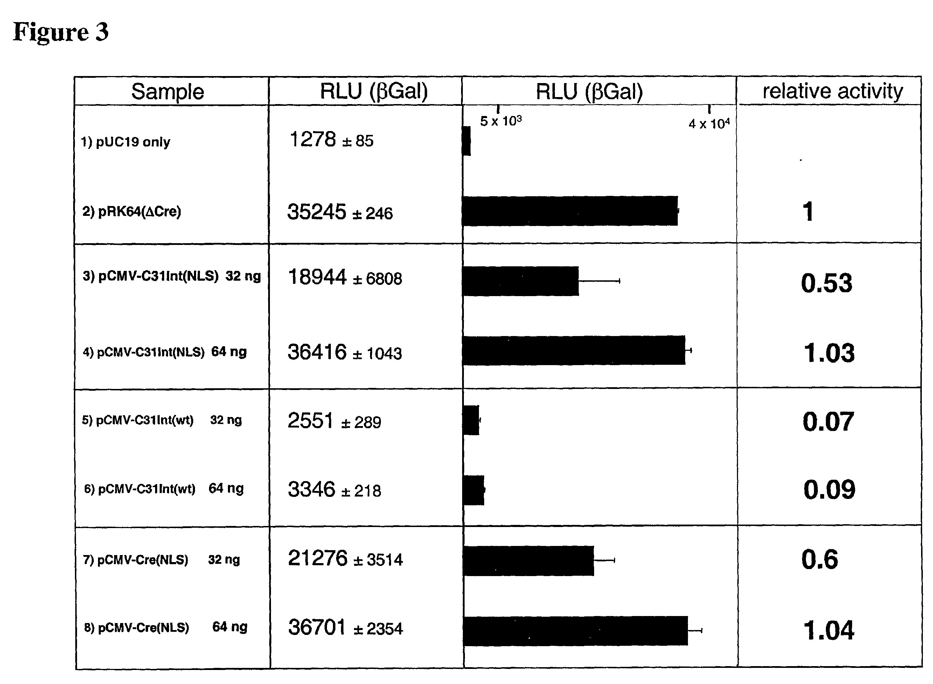

Figure 3 shows results of transient transfections of recombinase vectors into a stable reporter cell line.
   All transfections were performed with a NIH 3T3 derived clone containing stably integrated copies of the pRK64 recombination substrate vector. Either 32 ng or 64 ng of the recombinase expression plasmids pCMV-C31-Int(NLS) (samples 3-4), pCMV-C31-Int(wt) (samples 5-6) or pCMV-Cre (samples 7-8). Negative control: transfection with pUC19 alone (sample 1). Positive control: transfection with the Cre-recombined reporter pRK64(ΔCre) (sample 2).
   The vertical rows show the mean values and standard deviation of relative light units obtained from lysates with the assay for β-galactosidase (RLU(β-Gal)) and the relative activity of the various recombinases as compared to the positive control defined as 1.

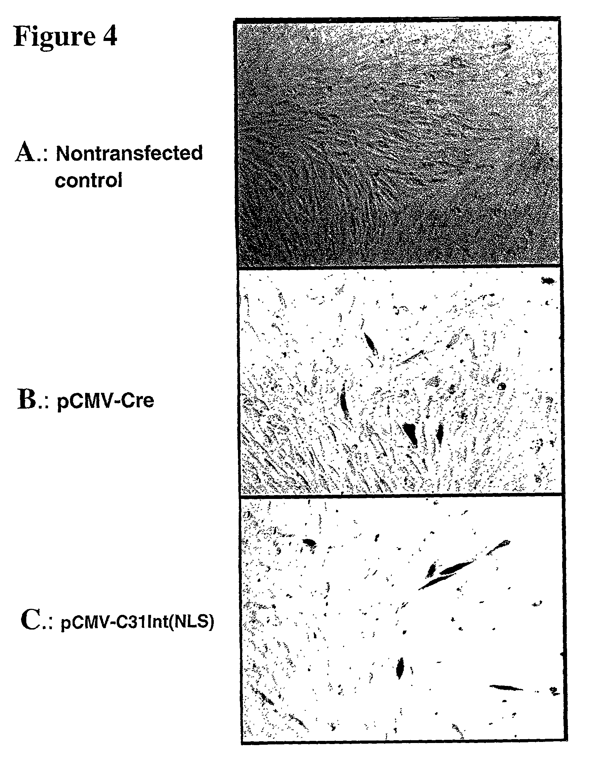

Figure 4 shows the in situ detection of β-galactosidase in 3T3(pRK64)-3 cells transfected with recombinase expression vectors.
   The Cre and C31-Int recombinase reporter cell line 3T3(pRK64)-3 was either not transfected with DNA (A), transfected with the Cre expression vector pCMV-Cre (B) or with the C31-Int expression vector pCMV-C31-Int(NLS). Two days after tranfection the cells were fixed and incubated with the histochemical X-Gal assay which develops a blue stain in β-galactosidase positive cells indicating recombinase mediated activation of the reporter gene.

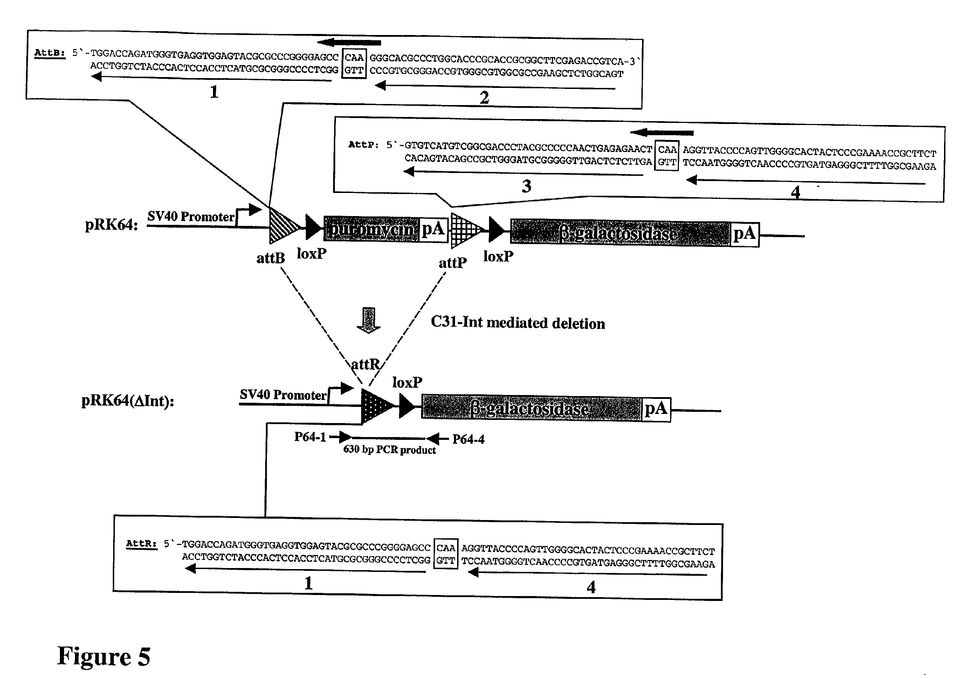

Figure 5 shows the test vector for C31-Int mediated deletion, pRK64, and the expected product of deletion, pRK64(ΔInt).
   Plasmid pRK64 contains the 1.1 kb cassette of the coding region of the puromycin resistance gene and a polyadenylation signal, which is flanked 5' by the 84 bp attB and 3' by the 84 bp attP recognition site (large triangles) of C31-Int. These attB and attP sites are oriented in the same way to each other (thick black arrows) which is used by the ΦX31 phage to integrate into the bacterial genome. In addition, the cassette is flanked by two Cre recombinase recognition (loxP) sites in the same orientation (black small triangles). For better orientation the half sites of the att sequences are labelled by a direction (thin arrow) and numbered 1-4. The 3 bp sequence within the att sites at which recombination occurs is framed by a box. The positions at which the PCR primers P64-1 and P64-4 hybridise to the pRK64 vector are indicated by arrows, pointing into the 3'direction of both oligonucleotides.
   PRK64(ΔInt) depicts the deletion product expected from the C31-Int mediated recombination between the att sites of pRK64. The recombination between a pair of attB/attP sites generates an attR site remaining on the parental DNA molecule while the puromycin cassette is excised. In this configuration the primers P64-1 and P64-4 will amplify a PCR product of 630 bp from pRK64(ΔInt).

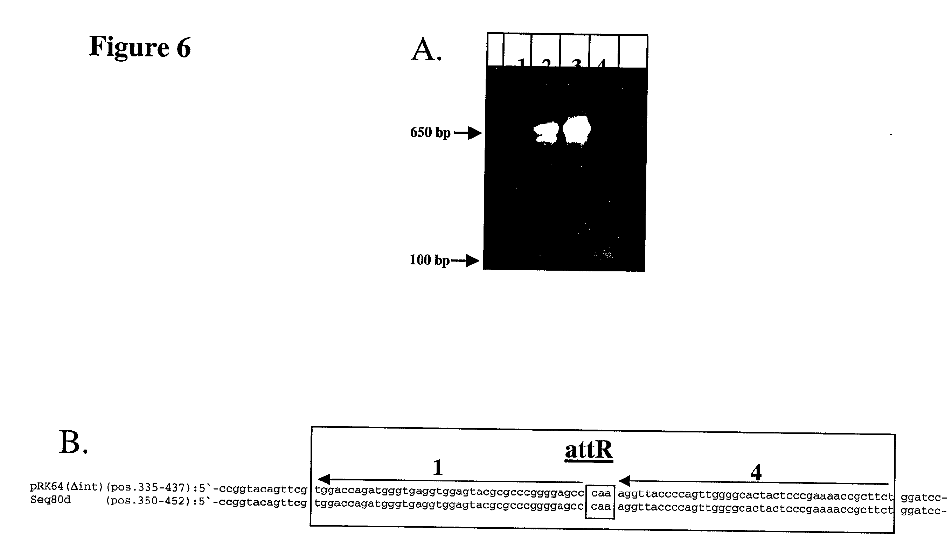

Figure 6 shows PCR products generated with the primers P64-1 and P64-4 and a sequence comparison of the PCR product.
   A: Analysis of PCR products on an agarose gel from PCR reactions using the Primers P64-1 and P64-4 on DNA extracted from MEF5-5 cells transfected 2 days before with plasmid pRK64 alone (lane 4), with pRK64 + CMV-Cre (lane 3), with pRK64 + pCMV-C31-Int(wt) (lane 2), and from a control reaction which did not contain cellular DNA (lane 1). The product with an apparent size around 650 bp, as compared to the size marker used, from lane 2 was excised from the agarose gel and purified. The PCR product was cloned into a sequencing plasmid vector and gave rise to the plasmid pRK80d. The insert of this plasmid was sequenced using reverse primer (seq80d) and compared to the predicted sequence of the pRK64 vector after C31-Int mediated deletion of the att flanked cassette, pRK64(ΔInt). The cloned PCR product shows a 100% identity with the predicted attR sequence after deletion. The generated attR site is shown in a box, with the same sequence designation used in Figure 5. The nucleotide positions (pos.) of the compared sequences pRK64(ΔInt) and Seq80d are indicated.

The present invention is further illustrated by the following Examples which are, however not to be construed as to limit the invention.

### Examples

### Example 1

As compared to Cre recombinase the wildtype form of C31-Int exhibits a significantly lower recombination activity in mammalian cells which falls in the range of 10 - 40% of Cre, depending on the assay system used (see below). As a measure which may increase C31-Int efficiency in eukaryotic cells a mammalian expression vector for a fusion protein of C31-Int with a peptide was designed which is recognised by the nuclear import machinery. The recombination efficiency obtained by this modified C31-Int recombinase in mammalian cells was compared side by side to the unmodified (wildtype) form of C31-Int and to Cre recombinase. For the quantification of recombinase activities the expression vectors were transiently introduced into a mammalian cell line together with a reporter vector which contains C31-Int and Cre target sites and leads to the expression of β-galactosidase upon recombnase mediated deletion of a vector segment flanked by recombinase recognition sites.

A. Plasmid constructions: Construction of the recombination test vector pRK64: first an attB site (Thorpe et al. Proc. Natl. Acad. Sci. USA, 95, 5505 - 5510 (1998)), generated by the annealing of the two synthetic oligonucleotides C31-4 (SEQ ID NO:1) and C31-5 (SEQ ID NO:2), was ligated into the BstBI restriction site of the vector PSV-Pax1 (Buchholz et al., Nucleic Acids Res., 24, 4256-4262 (1996)), 5' of its puromycin resistance gene and loxP site, giving rise to plasmid pRK52. The sequence and orientation of the cloned attB site was confirmed by DNA sequence analysis. Next, an attP site site (Thorpe et al. Proc. Natl. Acad. Sci. USA, 95, 5505 - 5510 (1998)), generated by the annealing of the two synthetic oligonucleotides C31-6 (SEQ ID NO:3) and C31-7-2 (SEQ ID NO:4), was ligated into the BamHI restriction site of plasmid pRK52, downstream of the puromycin resistance gene and loxP site, giving rise to plasmid pRK64 (SEQ ID NO: 5). The sequence and orientation of the attP site was confirmed by DNA sequence analysis. In pRK64 the newly cloned attB (position 348 - 431) and attP (position 1534 - 1617) sites are in the same orientation flanking the puromycin resistance gene and the SV40 early polyadenylation sequence. The attB and attP sites are followed by loxP sites in the same orientation (positions 435 - 469 and 1624 - 1658). The puromycin cassette is transcribed from the SV40 early enhancer/promoter region and followed by the coding region for E. coli β-galactosidase and the SV40 late region polyadenylation sequence.

Construction of C31-Int expression vectors: First the C31-Int gene of phage ΦC31 was amplified by PCR from phage DNA (DSM-49156, DSMZ-Deutsche Sammlung von Mikroorganismen und Zellkulturen GmbH, Mascheroder Weg 1b, D-38124 Braunschweig, Germany) using the primers C31-1 (SEQ ID NO:6)and C31-3 (SEQ ID NO:7). The ends of the PCR product were digested with NotI and the product was ligated into plasmid pBluescript II KS, opened with NotI, giving rise to plasmid pRK40. The DNA sequence of the 1.85 kb insert was determined and found to be identical to the published C31-Int gene (Kuhstoss et al., J. Mol. Biol. 222, 897-908 (1991)), except for an error in the stop codon. This error was repaired by PCR amplification of a 300 bp fragment from plasmid pRK40 using the primers C31-8 (SEQ ID NO:8) and C31-9 (SEQ ID NO:9), which provide a corrected Stop codon. The ends of this PCR fragment were digested with Eco47III and XhoI and the fragment ligated into plasmid pRK40, opened with Eco47III and XhoI to remove the fragment containing the defective stop codon. The resulting plasmid, pRK55, contains the correct C31-Int gene as confirmed by DNA sequence analysis.

The C31-Int gene was isolated from pRK55 as 1.85 kb fragment by digestion with NotI and XhoI and ligated into the generic mammalian expression vector pRK50 (see below), opened with NotI and XhoI, giving rise to the C31-Int expression vector pCMV-C31-Int(wt). pCMV-C31-Int(wt) (SEQ ID NO:10) contains a 700 bp Cytomegalovirus immediated early gene promoter (position 1 - 700), a 270 bp hybrid intron (position 701 - 970), the C31-Int gene (position 978 - 2819), and a 189 bp synthetic polyadenylation sequence (position 2831 - 3020). For the construction of pCMV-C31-Int(NLS), the 3'-end of the C31-Int gene was amplified from pCMV-C31-Int(wt) as a 300 bp PCR fragment using the primers C31-8 (SEQ ID NO:8) and C31-2-2 (SEQ ID NO:11). Primer C31-2-2 modifies the 3'-end of the wildtype C31-Int gene such that the stop codon is replaced by a sequence of 21 basepairs coding for the SV40 T-antigen nuclear localisation sequence of 7 amino acids (Prolin-Lysin-Lysin-Lysin-Arginin-Lysin-Valin) (Kalderon et. al, Cell, 39, 499 - 509 (1984)), followed by a new stop codon. The ends of this 300 bp PCR fragment were digested with with Eco47III and XhoI and the fragment ligated into plasmid pCMV-C31-Int(wt), opened with Eco47III and XhoI, to replace the 3'-end of the wildtype C31-Int gene, resulting in the plasmid pCMV-C31-Int(NLS). The identity of the new gene segment was verified by DNA sequence analysis. pCMV-C31-Int(NLS) (SEQ ID NO:12) contains a 700 bp Cytomegalovirus immediated early gene promoter (position 12 - 711), a 270 bp hybrid intron (position 712 - 981), the modified C31-Int gene (position 989 - 2851), and a 189 bp synthetic polyadenylation sequence (position 2854 - 3043).

To generate the Cre expression plasmid pCMV-Cre (SEQ ID NO:13), the coding sequence of Cre recombinase (Sternberg et al., J. Mol. Biol., 187, 197 - 212 (1986)) with a N-terminal fusion of the 7 amino acid SV40 T-antigen NLS (see above) was recovered from plasmid pgk-Cre and cloned into the NotI and XhoI sites of plasmid pRK50. PRK50 (SEQ ID NO:14) is a generic expression vector for mammalian cells based on the cloning vector pNEB193 (New England Biolabs Inc, Beverly, MA, USA). PRK50 was build by insertion into pNEB193 of a 700 bp Cytomegalovirus immediated early gene (CMV-IE) promoter (position 1-700) from plasmid pIREShyg (GenBank#U89672; Clontech Laboratories Inc, Palo Alto, CA, USA), a synthetic 270 bp hybrid intron (position 701-970), consisting of a adenovirus derived splice donor and an IgG derived splice acceptor sequence (Choi et al., Mol. Cell. Biol., 11, 3070 - 3074 (1991)), and a 189 bp synthetic polyadenylation sequence (position 1000-1188) build from the polyadenylation consensus sequence and 4 MAZ polymerase pause sites (Levitt et al., Genes&Dev., 3, 1019 - 1025 (1989); The EMBO J. 13, 5656 - 5667 (1994)). The positive control plasmid pRK64(ΔCre) (SEQ ID NO:15) was generated from pRK64 by transformation into the Cre expressing E. coli strain 294-Cre (Buchholz et al., Nucleic Acids Res., 24, 3118 - 3119 (1996)).

One of the transformed subclones was confirmed for the Cre mediated deletion of the loxP-flanked cassette by restriction mapping and further expanded. Plasmid pUC19 is a cloning vector without eukaryotic control elements used to equalise DNA amounts for transfections (GenBank#X02514; New England Biolabs Inc, Beverly, MA, USA). All plasmids were propagated in DH5α E.coli cells (Life Technologies GmbH, Karlsruhe, Germany) grown in Luria-Bertani medium and purified with the plasmid DNA purification reagents "Plasmid-Maxi-Kit" (Quiagen GmbH, Hilden, Germany) or "Concert high purity plasmid purification system" (Life Technologies GmbH, Karlsruhe, Germany). Following purification the plasmid DNA concentrations were determined by absorption at 260 nm and 280 nm in UVette cuvettes (Eppendorf-Netheler-Hinz GmbH, Hamburg, Germany) using a BioPhotometer (Eppendorf-Netheler-Hinz GmbH, Hamburg, Germany) and the plasmids were diluted to the same concentration; finally these were confirmed by separation of 10 ng of each plasmid on an Ethidiumbromide-stained agarose gel.

B. Cell culture and transfections: Chinese hamster ovary (CHO) cells (Puck et al., J. Exp. Med., 108, 945 (1958)) were obtained from the Institute for Genetics (University of Cologne, Germany) as a population adapted to growth in DMEM medium. The cells were grown in DMEM/Glutamax medium (Life Technologies) supplemented with 10% fetal calf serum at 37°C, 10% CO₂ in humid atmosphere and passaged upon trypsinisation.

One day before transfection 10⁶ cells were plated into a 48-well plate (Falcon). For the transient transfection of cells with plasmids each well received into 250 ml of medium a total amount of 300 ng supercoiled plasmid DNA complexed before with the FuGene6 transfection reagent (Roche Diagnostics GmbH, Mannheim, Germany) according to the manufacturers protocol. Each 300 ng DNA preparation (Fig.2 sample 4-9) contained 50 ng of the Luciferase expression vector pUHC13-1 (Gossen et al., Proc Natl Acad Sci USA., 89 5547-5551 (1992)), 50 ng of the substrate vector pRK64, 0.5 ng or 1 ng of one the recombinase expression vectors pCMV-C31Int(wt), pCMV-C31Int(NLS) or pCMV-Cre and 199 ng or 199.5 ng of pUC19 plasmid, except for the controls which received 50 ng of pUHC13-1 together with 50 ng of pRK64 (sample 3) or pRK64(Δcre) (sample 2) and 200 ng pUC19, or 50 ng pUHC13-1 with 250 ng pUC19 (sample 1). As the C31-Int expression vectors are 15% larger in size than pCMV-Cre and the same amounts of DNA of the three plasmids were used for transfection, the samples with C31-Int vectors received 15% less plasmid molecules as compared to the samples with Cre expression vector. The β-galactosidase values from C31-Int transfected samples by this value were not corrected and thus is a slight underestimation of the calculated C31-Int activities. For each sample to be tested four individual wells were transfected. One day after the addition of the DNA preparations each well received additional 250 ml of growth medium. The cells of each well were lysed 48 hours after transfection with 100 ml Lysate reagent supplemented with protease inhibitors (Roche Diagnostics). The lysates were centrifuged and 20 ml were used to determine the β-galactosidase activities using the β-galactosidase reporter gene assay (Roche Diagnostics) according to the manufacturers protocol in a Lumat LB 9507 luminometer (Berthold). To measure Luciferase activity, 20ml lysate was diluted into 250ml assay buffer (50mM glycylglycin, 5mM MgCl₂, 5mM ATP) and the relative light units (RLU) were counted in a Lumat LB 9507 luminometer after addition of 100 ml of a 1 mM Luciferin (Roche Diagnostics) solution. The mean value and standard deviation of the samples was calculated from the β-galactosidase and luciferase RLU values obtained from the four transfected wells of each sample.

C. Results: To set up an assay system for the measurement of C31-Int and Cre recombinase efficiency in mammalian cells the recombination substrate vector pRK64 shown in Figure 1D was first constructed. pRK64 contains a SV40 promoter region for expression in mammalian cells followed by a 1.1 kb cassette which consists of the coding region of the puromycin resistance gene and a polyadenylation signal sequence. This cassette is flanked at the 5'-end by the 84 bp attB and at the 3'-end by the 84 bp attP recognition site of C31-Int (Fig.1 and 5). These attB and attP sites are located on the same DNA molecule and oriented in a way to each other which allows the deletion of the flanked DNA segment. The same orientation of attB and attP sites is used naturally by the ΦX31 phage and the bacterial genome, leading to the integration of the phage genome when both sites are located on different DNA molecules (Thorpe et al., Proc. Natl. Acad. Sci. USA, 95, 5505 - 5510 (1998)). To measure C31-Int and Cre recombinase activities with the same substrate vector, pRK64 contains in addition two Cre recognition (loxP) sites in direct orientation next to the att sites. Since the att/lox-flanked cassette in plasmid pRK64 is inserted between the SV40 promoter and the coding region of the β-galactosidase gene, its presence inhibits β-galactosidase expression as the SV40 promoter derived transcripts are terminated at the polyadenylation signal of the puromycin gene. Plasmid pRK64 is turned into a β-galactosidase expression vector upon C31-Int or Cre mediated deletion of the att/lox-flanked puromycin cassette since the remaining single att and loxP site do not substantially interfere with gene expression.

For the expression of recombinases a mammalian expression vector was designed which contains the CMV immediate early promoter followed by a hybrid intron, the coding region of the recombinase to be tested, and an artificial polyadenylation signal sequence. The backbone sequence of the three recombinase expression vectors shown in Figure 1A-C is identical to each other except for the recombinase coding region. Plasmid pCMV-C31Int(wt) (Fig1A) contains the nonmodified (wildtype) 1.85 kb coding region of C31-Int as found in the genome of phage ΦC31 (Kuhstoss, et al., J. Mol. Biol. 222, 897-908 (1991)). Plasmid pCMV-C31Int(NLS) (Fig1B) contains a modified C31-Int gene coding for the full length C31-Int protein with a C-terminal extension of 7 amino acids derived from the SV40 virus large T antigen which serves as a nuclear localisation signal (NLS). Plasmid pCMV-Cre (Fig.1C) contains the 1.1 kb Cre coding region with an N-terminal fusion of the 7 amino acid NLS of the SV40 T-antigen. For Cre recombinase it has been shown that the N-terminal addition of the SV40 T-antigen NLS does not increase its recombination efficiency in mammalian cells (Le et al., Nucleic Acids Res., 27, 4703 - 4709 (1999)).

As a test system to compare the efficiency of the 3 recombinases the same amount of plasmid DNA of each of the recombinase expression vectors together with a fixed amount of the reporter plasmid pRK64 was transiently introduced into Chinese Hamster Ovary (CHO) cells. Thus, in this assay design the efficiency of the various recombinases on an extrachromosomal substrate introduced into the CHO cells was compared as a circular plasmid. Two days after transfection the cells from the various samples were lysed and the activity of β-galactosidase in the lysates was determined by a specific chemiluminescense assay and expressed in Relative Light Units (RLU)(β-Gal) (Fig. 2). In addition all samples contained a fixed amount of a Luciferase expression vector to control for the experimental variation of cell transfection and lysis. For this purpose the lysates of each sample were also tested for Luciferase activity with a specific chemiluminescense assay and the values expressed as Relative Light Units (RLU)(Luciferase) (Fig. 2). All transfection samples contained in addition varying amounts of the unrelated cloning plasmid pUC19 so that all samples were equalised to the same amount of plasmid DNA. As a positive control for β-galactosidase a derivative of the recombination reporter pRK64 was used in which the loxP flanked 1.1 kb cassette has been removed through Cre mediated recombination in E. coli giving rise to plasmid pRK64(ΔCre). As negative controls served samples which received the unrecombined reporter plasmid pRK64 but no recombinase expression vector as well as samples set up with the pUC19 plasmid alone.

To determine the relative efficiency of the tested recombinases the RLU values of β-galactosidase were divided individually for each sample by the RLU values obtained for Luciferase and multiplied with 10⁵. From the values of the four data points of each sample the mean value and standard deviation was calculated as an indicator of recombinase activity (Gal/Luc) (Fig.2). The relative activity of the tested recombinases was then compared to the positive control defined as an activity of 1.

As shown in Figure 2 the expression of Cre recombinase (samples 8 and 9) resulted in a 150 - 170-fold increase of β-galactosidase activity as compared to the negative control (sample 3), demonstrating the wide dynamic range of our test system. Each recombinase vector was tested using two different amounts of DNA for transfection (0.5 and 1ng/sample), which in the case of Cre resulted in 63% and 72% recombinase activity (samples 8 and 9) as compared to the positive control. These two values establish that the DNA amounts used are close to the test systems saturation for recombinase expression as the doubling of DNA amounts resulted only in a minor increase of recombinase activity.

In comparison to Cre, the expression of wildtype C31-Int resulted in a considerably lower recombinase activity of 23% and 30% (Fig. 2, samples 6 and 7) as compared to the positive control. These values represent 37% and 42% recombinase activity for wildtype C31-Int as compared to Cre recombinase (compare samples 6 to 8 and 7 to 9). Upon the expression of C31-Int fused with the C-terminal NLS (C31-Int(NLS)) values of 50% and 65% recombinase activity (samples 4 and 5) were obtained as compared to the positive control. The C31-Int(NLS) values represent 79% and 90% recombinase activity as compared to Cre recombinase (compare samples 4 to 8 and 5 to 9). Hence, C31-Int(NLS) exhibits a more than twofold increase of recombinase activity in comparison to C31-Int(wt) (compare samples 4 to 6 and 5 to 7).

In conclusion, upon the transient transfection of both, the recombinase expression and the recombination substrate vectors in the test system shown in the present application a more than twofold activity increase of the ΦC31 Integrase by the addition of the SV40 T antigen NLS signal was observed. As this signal sequence has been characterised to act as a nuclear localisation signal (Kalderon et. al, Cell, 39, 499 - 509 (1984)) it is concluded that the efficiency increase of C31-Int(NLS) is the result of the improved nuclear accumulation of this recombinase. In particular, it could be shown that C31-Int(NLS) recombines extrachromosomal DNA in mammalian cells almost as efficient as the widely used Cre recombinase and thus provides an additional or alternative recombination system of highest activity. The efficiency increase of C31-Int(NLS) as compared to its wildtype form is regarded as an invention of substantial use for biotechnology.

### Example 2

As demonstrated in example 1 C31-Int recombinase with the C-terminal fusion of the SV40 T-antigen NLS (C31-Int(NLS)) shows in mammalian cells a recombination activity comparable to Cre recombinase on an extrachromosomal plasmid vector. It was further tried to test whether C31-Int(NLS) exhibits a similar activity on a recombination substrate which is chromosomally integrated into the genome of mammalian cells. This question is critical for the use of a recombination system for genome engineering as it is possible that a recombinase may act efficiently on extrachromosomal substrates but is impaired if the recognition sites are part of the mammalian chromatin. To characterise the recombination activity of C31-Int(NLS) on a chromosomal substrate the pRK64 reporter plasmid (Fig. 1D) was stably integrated, containing a pair of loxP and att sites, into the genome of a mammalian cell line. One of the stable transfected clones was choosen for further analysis and was transiently transfected with recombinase expression vectors coding for C31-Int(NLS), C31-Int(wt) or Cre recombinase. As used in example 1, the activity of β-galactosidase derived from the pRK64 vector recombined in these cells was taken as a measure of recombination efficiency.

A. Plasmid constructions: all plasmids used and their purification are described in example 1.

B. Cell culture and transfections: To generate a stably transfected C31-Int reporter cell line 2.5 x 10⁶ NIH-3T3 cells (Andersson et al., Cell, 16, 63-75 (1979); DSMZ#ACC59; DSMZ-Deutsche Sammlung von Mikroorganismen und Zellkulturen GmbH, Mascheroder Weg 1b, D-38124 Braunschweig, Germany) were electroporated with 5 µg pRK64 plasmid DNA linearised with ScaI and plated into 10cm petri dishes. The cells were grown in DMEM/Glutamax medium (Life Technologies) supplemented with 10% fetal calf serum at 37°C, 10% CO₂ in humid atmosphere and passaged upon trypsinisation. Two days after tranfection the medium was supplemented with 1mg/ml of puromycin (Calbiochem) for the selection of stable integrants. Upon the growth of resistant colonies these were isolated under a stereomicroscope and individually expanded in the absence of puromycin. To demonstrate stable integration of the transfected vector, genomic DNA of puromycin resistant clones was prepared according to standard methods and 5-10 µg were digested with EcoRV: Digested DNA was separated in a 0.8% agarose gel and transferred to nylon membranes (GeneScreen Plus, NEN DuPont) under alkaline conditions for 16 hours. The filter was dried and hybridised for 16 hours at 65°C with a probe representing the 5' part of the E. coli β-galactosidase gene (1.25 kb NotI - EcoRV fragment of plasmid CMV-β-pA (R. Kühn, unpublished). The probe was radiolabelled with P32-marked α-dCTP (Amersham) using the Megaprime Kit (Amersham). Hybridisation was performed in a buffer consisting of 10% Dextranesulfate, 1% SDS, 50mM Tris and 100mM NaCL, pH7.5). After hybridisation the filter was washed with 2x SSC/1%SDS and exposed to BioMax MS1 X-ray films (Kodak) at - 80°C. Transfections transfections of the selected clone 3T3(pRK64)-3 with plasmid DNAs and the measurement of β-galactosidase activities in lysates were essentially performed as described in example 1 for CHO cells, except that 32ng or 64ng of the recombinase expression plasmids and 218 or 186 ng of pUC19 plasmid were used and the pRK64 plasmid was omitted from all samples.

### C. Histochemical detection of β-galactosidase activity in transfected 3T3(pRK64)-3 cells

To directly demonstrate β-galactosidase expression in recombinase transfected cells, 10⁴ 3T3(pRK64)-3 cells were plated one day before transfection into each well of a 48-well tissue culture plate (Falcon). For the transient transfection of cells with plasmids each well received into 250 µl of medium a total amount of 150 ng supercoiled plasmid DNA complexed before with the FuGene6 transfection reagent (Roche Diagnostics GmbH, Mannheim, Germany) according to the manufacturers protocol. Each 150 ng DNA preparation contained 50 ng of the recombinase expression vector pCMV-Cre or pCMV-C31Int(NLS) and 100ng of the pUC19 plasmid. After 2 days the culture medium was removed from the wells, the wells were washed once with phophate buffered saline (PBS), and the cells were fixed for 5 minutes at room temperature in a solution of 2% formaldehyde and 1% glutaraldehyde in PBS. Next, the cells were washed twice with PBS and finally incubated in X-Gal staining solution for 24 hours at 37°C (staining solution: 5 mM K3(Fe(CN)6), 5mM K4(Fe(CN)6), 2mM MgCl2, 1mg/ml X-Gal (BioMol) in PBS) until photographs were taken.

### D. Results

To generate a mammalian cell clone with a stable genomic integration of the C31-Int and Cre recombinase reporter plasmid pRK64, the murine fibroblast cell line NIH-3T3 was electroporated with linearised pRK64 DNA (Fig.1D; see also example 1) and subjected to selection in puromycin containing growth medium. Plasmid pRK64 contains in between the pair of loxP and att sites the coding region of the puromycin resistance gene expressed from the SV40-IE promoter. Thirtysix puromycin resistant clones were isolated and the genomic DNA of 19 clones was analysed for the presence and copy number of the pRK64 DNA. Three clones, which apparently contain 2 - 4 copies of pRK64, were further characterised on the single cell level for the expression of β-galactosidase upon transient transfection with the Cre expression vector pCMV-Cre (Fig. 1C). The cell clone with the highest proportion of β-galactosidase positive cells, 3T3(pRK64)-3, was selected as most useful for the planned studies on C31-Int and Cre recombinase efficiency.

To compare the efficiency of wildtype C31-Int (C31-Int(wt)), C31-Int(NLS) and Cre recombinases 32ng or 64 ng of the recombinase expression vectors pCMV-C31Int(wt), pCMV-C31Int(NLS) or pCMV-Cre (Fig. 1 A-C) together with the unrelated cloning plasmid pUC19 were transiently introduced into 3T3(pRK64)-3 cells, such that all samples contained the same amount of plasmid DNA. As a positive control for β-galactosidase a derivative of the recombination reporter pRK64 was used in which the loxP flanked 1.1 kb cassette has been removed in E. coli through Cre mediated recombination giving rise to plasmid pRK64(ΔCre). As a negative control a sample prepared with the pUC19 plasmid alone was used. Two days after transfection the cells from the various samples were lysed and the activity of β-galactosidase in the lysates was determined by a specific chemiluminescense assay and expressed in Relative Light Units (RLU)(β-Gal) (Fig. 3). From the values of the four data points of each sample the mean value and standard deviation was calculated as an indicator of recombinase activity (Fig.3). The relative activity of the tested recombinases was then compared to the positive control defined as an activity of 1.

As shown in Figure 3 the expression of Cre recombinase (samples 7 and 8) resulted in a 17 - 29-fold increase of β-galactosidase activity as compared to the negative control (sample 1), demonstrating the dynamic range of the test system used. Each recombinase vector was tested using two different amounts of DNA for transfection (32 ng and 64 ng/sample), which in the case of Cre resulted in 60 % and 104 % recombinase activity (samples 7 and 8) as compared to the positive control. These two values establish that the DNA amounts used are close to the linear scale of the test systems ability for recombinase expression as the twofold increase of the amount of DNA also resulted almost in the double recombinase activity.

In contrast to Cre, the expression of wildtype C31-Int (Fig. 3, samples 5 and 6) resulted in a considerably lower recombinase activity of 7 % and 9 % as compared to the positive control. These values represent 11 % and 9 % recombinase activity for wildtype C31-Int as compared to Cre recombinase (compare samples 5 to 7 and 6 to 8). Upon the expression of C31-Int(NLS) values of 53 % and 103 % recombinase activity (samples 3 and 4) were obtained as compared to the positive control. The C31-Int(NLS) values represent 88 % and 99 % recombinase activity as compared to Cre recombinase (compare samples 3 to 7 and 4 to 8). Hence, C31-Int(NLS) exhibited an 8-fold higher activity than C31-Int(wt) at 32 ng plasmid DNA (Fig.3, compare samples 3 and 5) and an 11-fold higher activity than C31-Int(wt) at 64 ng plasmid DNA (Fig.3, compare samples 4 and 6).

In addition it was aimed to directly demonstrate in situ the expression of β-galactosidase in 3T3(pRK64)-3 cells after transfection with Cre or C31-Int(NLS) recombinase plasmid. Two days after transfection the cells were fixed in situ and incubated with the histochemical X-Gal assay which detects β-galactosidase positive cells by a blue precipitate. As shown in Figure 4 stained cells were found at a comparable frequency in the samples transfected with the Cre or C31-Int(NLS) expression vectors but not in the nontransfected control. This result confirms that the β-galactosidase activities measured by chemiluminescense upon recombinase transfection (Fig. 3) results from a population of individual, recombined reporter cells

In conclusion, upon the transient transfection of recombinase expression vectors into a cell line with a genomic integration of the recombination substrate vector, a 8 - 11-fold activity increase of the ΦC31 Integrase by the fusion with the SV40 T-antigen NLS signal was used. As this signal sequence has been characterised to act as a nuclear localisation signal (Kalderon et. al, Cell, 39, 499 - 509 (1984)), it was concluded that the dramatic efficiency increase of C31-Int(NLS) is the result of the improved nuclear accumulation of this recombinase. The approximately tenfold activity increase of C31-Int(NLS) upon expression in a cell line with a genomic integration of the substrate vector is considerably higher than the activity increase found upon the transient expression of both vectors (see example 1). Thus, a substrate vector integrated into the chromatin of a mammalian cell may pose more stringent requirements on recombinase activity to be recombined as compared to an extrachromosomal substrate.

The dramatic activity increase of C31-Int(NLS), as compared to its wildtype form, on a stable integrated substrate in mammalian cells is an invention of significant practical use as this recombinase is as efficient as the widely used Cre/loxP system; thus, C31-Int(NLS) provides an additional or alternative recombination system of highest activity.

### Example 3

To demonstrate that the increase in β-galactosidase activity obtained by the cotransfection of a C31-Int expression vector and the reporter vector pRK64 into mammalian cells is in fact the result of recombinase mediated deletion, one of the recombination products was detected by a specific polymerase chain reaction (PCR). The amplified PCR product was cloned and its sequence determined. The obtained sequence confirms that C31-Int mediated deletion of the test vector occurs in a mammalian cell line and that the recombination occurs at the known breakpoint within the attB and attP sites.

A. Plasmid constructions: The construction of plasmids pRK64, pCMV-Cre and pCMV-C31-Int(wt) is described in Example 1. To simulate the recombination of pRK64 by C31-Int, the sequence between the CAA motives of the att sites (boxed in Fig.5) was deleted from the computerfile of pRK64, giving rise to the sequence of pRK64(ΔInt) (SEQ ID NO:16).

B. Transfection of Cells and PCR amplification: MEF5-5 mouse fibroblasts (Schwenk et al., 1998) (20000 cells per well of a 12 well plate (Falcon)) were transfected with 0.5 µg pRK64 alone or together with 250 ng pCMV-Int(wt) or pCMV-Cre using the FuGene6 transfection reagent following the manufacturers protocol (Roche Diagnostics). After 2 days DNA was extracted from these cells according to standard methods and used for PCR amplification with Primers P64-1 (SEQ ID NO:17; complementary to position 111-135 of pRK64(ΔInt)) and P64-4 (SEQ ID NO:18; complementary to position 740-714 of pRK64(ΔInt)) using the Expand High Fidelity PCR kit (Roche Diagnostics). PCR products were separated on a 0.8% agarose gel, extracted with the QuiaEx kit (Quiagen) and cloned into the pCR2.1 vector using the TA cloning kit (Invitrogen) resulting in plasmid pRK80d. The sequence of its insert, seq80d (SEQ ID NO:19), was determined using the reverse sequencing primer and standard sequencing methods (MWG Biotech).

For the measurement of β-galactosidase activity the cells were lysed 2 days after transfection and the β-galactosidase activities were determined with the β-galactosidase reporter gene assay (Roche Diagnostics) as described in example 1.

C. Results: As a test vector for C31-Int mediated DNA recombination plasmid pRK64 was used which contains the 1.1 kb coding region of the puromycin resistance gene flanked 5' by the 84 bp attB and 3' by the 84 bp attP recognition site of C31-Int (Fig.5). These attB and attP sites are located on the same DNA molecule and oriented in a way to each other which allows the deletion of the att-flanked DNA segment. The same orientation of attB and attP sites is used naturally by the ΦX31 phage and the bacterial genome for the integration of the phage genome when both sites are located on different DNA molecules (Thorpe et al., Proc. Natl. Acad. Sci. USA, 95, 5505 - 5510 (1998)). As a positive control, vector pRK64 contains in addition two Cre recombinase recognition (loxP) sites in direct orientation next to the att sites. Since the att-flanked DNA segment in plasmid pRK64 is inserted between a promoter active in mammalian cells and the β-galactosidase gene, its deletion can be measured by the increase of β-galactosidase activity. The expected product of C31-Int mediated deletion of plasmid pRK64 is shown in Figure 5, designated as pRK64(ΔInt). If the recombination between attB and attP occurs as described in bacteria (Thorpe et al., Proc. Natl. Acad. Sci. USA, 95, 5505-5510 (1998)), a single attR site is generated and left on the parental plasmid (Fig.5) while the flanked DNA is excised and contains an attL site. Beside the measurement of β-galactosidase activity, C31-Int mediated recombination of pRK64 can be directly detected on the DNA level by a specific polymerase chain reaction (PCR) using the primers P64-1 and P64-4 (Fig.5). These primers, located 5'of the attB site (P64-1) and 3' of the attP site are designed to amplify a PCR product of 630 bp lenght upon the C31-Int mediated recombination of pRK64. For the expression of C31-Int in mammalian cells plasmid pCMV-C31(wt) was used, which contains the CMV-IE-Promoter upstream of the C31-Int coding region followed by a synthetic polyadenylation signal (see Example 1 and Fig.1).
The recombination substrate vector pRK64 was transiently transfected into the murine fibroblast cell line MEF5-5 either alone, or together with the C31-Int expression vector pCMV-C31(wt), or together with an expression vector for Cre recombinase, pCMV-Cre. Two days after transfection half the cells of each sample was lysed and used to measure β-galactosidase activity by chemiluminescense and the other half was used for the preparation of DNA from the transfected cells for PCR analysis. The β-galactosidase levels of the 3 samples were found as following (expressed as relative light units (RLU) with standard deviation (SD) of the β-galactosidase assay):

| Sample | RLU (SD) |
|---|---|
| 1) pRK64 | 692 ± 5 |
| 2) pRK64 + pCMV-Cre | 8527 ± 269 |
| 3) pRK64 + pCMV-C31(wt) | 1288 ± 93 |

As the coexpression of the test vector pRK64 together with the C31-Int expression vector in sample 3 leads to a significant increase of β-galactosidase activity as compared to pRK64 alone, this result suggests that pRK64 is recombined by C31-Int as anticipated in Figure 5.

Next, cellular DNA was prepared from the three samples and tested for the occurrence of the expected Cre or C31-Int generated deletion product by PCR using primers P64-1 and P64-4 for amplification. As shown in Figure 6 an amplification product of the expected size was found only in the samples cotransfected with the Cre or C31-Int recombinase expression vectors (Fig.6A, lane3, lane 4). The PCR products amplified from pRK64 recombined by C31-Int or Cre are of the same size but should be recombined via the attB/P, or loxP sites respectively.
To prove that the PCR product found after cotransfection of plasmids pRK64 and pCMV-C31(wt) represents in fact the deletion product of C31-Int mediated recombination, this DNA fragment was cloned into a plasmid vector and its DNA sequence determined. One clone, pRK80d was analysed and its sequence showed exactly the sequence of an attR site as expected from C31-Int mediated deletion of pRK64 (Fig.6B, compare to Fig.5).

In conclusion, this experiment demonstrates that C31-Int mediated deletion of a vector containing a pair of attB/attP sites occurs in a mammalian cell line and that the recombination occurs within the same 3 bp breakpoint region of attB and attP as found in bacteria (Thorpe et al., Proc. Natl. Acad. Sci. USA, 95, 5505 - 5510 (1998)). Thus, it was concluded that an increase of β-galactosidase activity observed by cotransfection of the pRK64 reporter vector and a C31-Int expression vector in mammalian cells truly reflects C31-Int recombinase activity.

## Claims

1. A fusion protein comprising
(a) an integrase domain being a phage ΦC31 integrase (C31-Int) protein or a mutant thereof, and
(b) a signal peptide domain being linked to (a) and directing the nuclear import of said fusion protein into eucaryotic cells.

2. The fusion protein of claim 1, wherein the integrase domain is a C31-Int having the amino acid sequence shown in SEQ ID NO:21 or a C-terminal truncated form thereof, said truncated form of the C31-Int preferably comprising amino acid residues of 306 to 613 of SEQ ID NO:21.

3. The fusion protein of claims 1 or 2, wherein the
signal peptide domain is derived from yeast GAL4, SKI3, L29 or histone H2B proteins, polyoma virus large T protein, VP1 or VP2 capsid protein, SV40 VP1 or VP2 capsid protein, Adenovirus E1a or DBP protein, influenza virus NS1 protein, hepatitis virus core antigen or the mammalian lamin, c-myc, max, c-myb, p53, c-erbA, jun, Tax, steroid receptor or Mx proteins, SV40 T-antigen or other proteins with known nuclear localisation and preferably is derived from the SV40 T-antigen.

4. The fusion protein according to any one of claims 1 to 3, wherein the signal peptide domain
(i) has a length of 5 to 74, preferably 7 to 15 amino acid residues, and/or
(ii) comprises a segment of 6 amino acid residues having at least 2 positively charged basic amino acid residues, said basic amino acid residues being preferably selected from lysin, arginin and histidine.

5. The fusion protein of claim 3 or 4, wherein the signal peptide domain comprises a peptide sequence selected from a sequence shown in SEQ ID NOs:24 to 53, preferably the signal peptide comprises the amino acid sequence Pro-Lys-Lys-Lys-Arg-Lys-Val (SEQ ID NO:53).

6. The fusion protein according to any one of claims 1 to 5, wherein the signal peptide domain is linked to the N-terminal or C-terminal of the integrase domain or is integrated into the integrase domain, preferably the signal peptide domain is linked to the C-terminal of the integrase domain.

7. The fusion protein according to any one of claims 1 to 6 wherein the signal peptide domain is linked to the integrase domain directly or through a linker peptide, said linker preferably having 1 to 30 essentially neutral amino acid residues.

8. The fusion protein of claim 1 comprising the amino acid sequence shown in SEQ ID NO:23.

9. A DNA coding for the fusion protein according to any one of claims 1 to 8.

10. A vector containing the DNA as defined in claim 9.

11. A microorganism containing the DNA of claims 9 and/or the vector of claim 10.

12. A process for preparing the fusion protein as defined in claims 1 to 8 which comprises culturing a microorganism as defined in claim 11.

13. Use of the fusion protein as defined in claims 1 to 8 to recombine DNA molecules containing C31-Int recognition sequences in eucaryotic cells.

14. A cell, preferably a mammalian cell containing the DNA sequence of claim 9 in its genome.

15. The cell of claim 14, also containing C31-Int recognition sequences in its genome.

16. Use of the cell of claim 14 or 15 for studying the function of genes and for the creation of transgenic organisms.

17. A transgenic organism, preferably a transgenic mammal containing the DNA sequence of claim 9 in its genome.

18. The transgenic organism of claim 17 also containing C31-Int recognition sequences in its genome.

19. Use of the transgenic organism of claim 17 or 18 for studying gene function at various developmental stages.

20. A method for recombining DNA molecules of cells or organisms containing C31-Int recognition sequences which comprises supplying the cells or organisms with a fusion protein as defined in claims 1 to 8 or with a DNA sequence of claim 9 and/or a vector of claim 11 which are capable of expressing said fusion protein in the cell or organism.


SEQUENCE LISTING

<110> Artemis Pharmaceuticals GmbH

<120> Modified Intgrase of Phage PhiC31

<130> 002829ep/JH/ml

<140>
<141>

<160> 53

<170> PatentIn Ver. 2.1


<210> 1
<211> 86
<212> DNA
<213> Artificial Sequence

<220>
<223> Description of Artificial Sequence: primer

<400> 1
cgtgacggtc tcgaagccgc ggtgcgggtg ccagggcgtg cccttgggct ccccgggcgc 60
gtactccacc tcacccatct ggtcca 86


<210> 2
<211> 86
<212> DNA
<213> Artificial Sequence

<220>
<223> Description of Artificial Sequence:
      oligonucleotide C31-5

<400> 2
cgtggaccag atgggtgagg tggagtacgc gcccggggag cccaagggca cgccctggcc 60
cacgcaccgc ggcttcgaga ccgtca 86


<210> 3
<211> 90
<212> DNA
<213> Artificial Sequence

<220>
<223> Description of Artificial Sequence:
      oligonucleotide C31-6

<400> 3
gatccagaag cggttttcgg gagtagtgcc ccaactgggg taacctttga gttctctcag 60
ttgggggcgt agggtcgccg acatgacacg 90


<210> 4
<211> 90
<212> DNA
<213> Artificial Sequence

<220>
<223> Description of Artificial Sequence:
      oligonucleotide C31-7-2

<400> 4
gatccgtgtc atgtcggcga ccctacgccc ccaactgaga gaactcaaag gttaccccag 60
ttggggcact actcccgaaa accgcttctg 90

<210> 5
<211> 7438
<212> DNA
<213> Artificial Sequence

<220>
<223> Description of Artificial Sequence: vector pRK64

<400> 5
cgtcatcacc gaaacgcgcg aggcagctgt ggaatgtgtg tcagttaggg tgtggaaagt 60
ccccaggctc cccagcaggc agaagtatgc aaagcatgca tctcaattag tcagcaacca 120
ggctccccag caggcagaag tgtgcaaagc atgcatctca attagtcagc aaccatagtc 180
ccgcccctaa ctccgcccat cccgccccta actccgccca gttccgccca ttctccgccc 240
catggctgac taattttttt tatttatgca gaggccgagg ccgcctcggc ctaggaacag 300
tcgacgacac tgcagagacc tacttcacta acaaccggta cagttcgtgg accagatggg 360
tgaggtggag tacgcgcccg ggagcccaag ggcacgccct ggcacccgca ccgcggcttc 420
cgagaccgtc acgaataact tcgtatagca tacattatac gaagttataa gcttgcatgc 480
ctgcaggtcg gccgccacga ccggccggcc ggtgccgcca ccatcccctg acccacgccc 540
ctgacccctc acaaggagac gaccttccat gaccgagtac aagcccacgg tgcgcctcgc 600
cacccgcgac gacgtccccc gggccgtacg caccctcgcc gccgcgttcg ccgactaccc 660
cgccacgcgc cacaccgtcg acccggaccg ccacatcgag cgggtcaccg agctgcaaga 720
actcttcctc acgcgcgtcg ggctcgacat cggcaaggtg tgggtcgcgg acgacggcgc 780
cgcggtggcg gtctggacca cgccggagag cgtcgaagcg ggggcggtgt tcgccgagat 840
cggcccgcgc atggccgagt tgagcggttc ccggctggcc gcgcagcaac agatggaagg 900
cctcctggcg ccgcaccggc ccaaggagcc cgcgtggttc ctggccaccg tcggcgtctc 960
gcccgaccac cagggcaagg gtctgggcag cgccgtcgtg ctccccggag tggaggcggc 1020
cgagcgcgcc ggggtgcccg ccttcctgga gacctccgcg ccccgcaacc tccccttcta 1080
cgagcggctc ggcttcaccg tcaccgccga cgtcgagtgc ccgaaggacc gcgcgacctg 1140
gtgcatgacc cgcaagcccg gtgcctgacg cccgccccac gacccgcagc gcccgaccga 1200
aaggagcgca cgaccccatg gctccgaccg aagccgaccc gggcggcccc gccgaccccg 1260
cacccgcccc cgaggcccac cgactctaga ggatcataat cagccatacc acatttgtag 1320
aggttttact tgctttaaaa aacctcccac acctccccct gaacctgaaa cataaaatga 1380
atgcaattgt tgttgttaac ttgtttattg cagcttataa tggttacaaa taaagcaata 1440
gcatcacaaa tttcacaaat aaagcatttt tttcactgca ttctagttgt ggtttgtcca 1500
aactcatcaa tgtatcttat catgtctgga tccgtgtcat gtcggcgacc ctacgccccc 1560
aactgagaga actcaaaggt taccccagtt ggggcactac tcccgaaaac cgcttctgga 1620
tccataactt cgtatagcat acattatacg aagttatacc gggccaccat ggtcgcgagt 1680
agcttggcac tggccgtcgt tttacaacgt cgtgactggg aaaaccctgg cgttacccaa 1740
cttaatcgcc ttgcagcaca tccccctttc gccagctggc gtaatagcga agaggcccgc 1800
accgatcgcc cttcccaaca gttgcgcagc ctgaatggcg aatggcgctt tgcctggttt 1860
ccggcaccag aagcggtgcc ggaaagctgg ctggagtgcg atcttcctga ggccgatact 1920
gtcgtcgtcc cctcaaactg gcagatgcac ggttacgatg cgcccatcta caccaacgta 1980
acctatccca ttacggtcaa tccgccgttt gttcccacgg agaatccgac gggttgttac 2040
tcgctcacat ttaatgttga tgaaagctgg ctacaggaag gccagacgcg aattattttt 2100
gatggcgtta actcggcgtt tcatctgtgg tgcaacgggc gctgggtcgg ttacggccag 2160
gacagtcgtt tgccgtctga atttgacctg agcgcatttt tacgcgccgg agaaaaccgc 2220
ctcgcggtga tggtgctgcg ttggagtgac ggcagttatc tggaagatca ggatatgtgg 2280
cggatgagcg gcattttccg tgacgtctcg ttgctgcata aaccgactac acaaatcagc 2340
gatttccatg ttgccactcg ctttaatgat gatttcagcc gcgctgtact ggaggctgaa 2400
gttcagatgt gcggcgagtt gcgtgactac ctacgggtaa cagtttcttt atggcagggt 2460
gaaacgcagg tcgccagcgg caccgcgcct ttcggcggtg aaattatcga tgagcgtggt 2520
ggttatgccg atcgcgtcac actacgtctg aacgtcgaaa acccgaaact gtggagcgcc 2580
gaaatcccga atctctatcg tgcggtggtt gaactgcaca ccgccgacgg cacgctgatt 2640
gaagcagaag cctgcgatgt cggtttccgc gaggtgcgga ttgaaaatgg tctgctgctg 2700
ctgaacggca agccgttgct gattcgaggc gttaaccgtc acgagcatca tcctctgcat 2760
ggtcaggtca tggatgagca gacgatggtg caggatatcc tgctgatgaa gcagaacaac 2820
tttaacgccg tgcgctgttc gcattatccg aaccatccgc tgtggtacac gctgtgcgac 2880
cgctacggcc tgtatgtggt ggatgaagcc aatattgaaa cccacggcat ggtgccaatg 2940
aatcgtctga ccgatgatcc gcgctggcta ccggcgatga gcgaacgcgt aacgcgaatg 3000
gtgcagcgcg atcgtaatca cccgagtgtg atcatctggt cgctggggaa tgaatcaggc 3060
cacggcgcta atcacgacgc gctgtatcgc tggatcaaat ctgtcgatcc ttcccgcccg 3120
gtgcagtatg aaggcggcgg agccgacacc acggccaccg atattatttg cccgatgtac 3180

gcgcgcgtgg atgaagacca gcccttcccg gctgtgccga aatggtccat caaaaaatgg 3240
ctttcgctac ctggagagac gcgcccgctg atcctttgcg aatacgccca cgcgatgggt 3300
aacagtcttg gcggtttcgc taaatactgg caggcgtttc gtcagtatcc ccgtttacag 3360
ggcggcttcg tctgggactg ggtggatcag tcgctgatta aatatgatga aaacggcaac 3420
ccgtggtcgg cttacggcgg tgattttggc gatacgccga acgatcgcca gttctgtatg 3480
aacggtctgg tctttgccga ccgcacgccg catccagcgc tgacggaagc aaaacaccag 3540
cagcagtttt tccagttccg tttatccggg caaaccatcg aagtgaccag cgaatacctg 3600
ttccgtcata gcgataacga gctcctgcac tggatggtgg cgctggatgg taagccgctg 3660
gcaagcggtg aagtgcctct ggatgtcgct ccacaaggta aacagttgat tgaactgcct 3720
gaactaccgc agccggagag cgccgggcaa ctctggctca cagtacgcgt agtgcaaccg 3780
aacgcgaccg catggtcaga agccgggcac atcagcgcct ggcagcagtg gcgtctggcg 3840
gaaaacctca gtgtgacgct ccccgccgcg tcccacgcca tcccgcatct gaccaccagc 3900
gaaatggatt tttgcatcga gctgggtaat aagcgttggc aatttaaccg ccagtcaggc 3960
tttctttcac agatgtggat tggcgataaa aaacaactgc tgacgccgct gcgcgatcag 4020
ttcacccgtg caccgctgga taacgacatt ggcgtaagtg aagcgacccg cattgaccct 4080
aacgcctggg tcgaacgctg gaaggcggcg ggccattacc aggccgaagc agcgttgttg 4140
cagtgcacgg cagatacact tgctgatgcg gtgctgatta cgaccgctca cgcgtggcag 4200
catcagggga aaaccttatt tatcagccgg aaaacctacc ggattgatgg tagtggtcaa 4260
atggcgatta ccgttgatgt tgaagtggcg agcgatacac cgcatccggc gcggattggc 4320
ctgaactgcc agctggcgca ggtagcagag cgggtaaact ggctcggatt agggccgcaa 4380
gaaaactatc ccgaccgcct tactgccgcc tgttttgacc gctgggatct gccattgtca 4440
gacatgtata ccccgtacgt cttcccgagc gaaaacggtc tgcgctgcgg gacgcgcgaa 4500
ttgaattatg gcccacacca gtggcgcggc gacttccagt tcaacatcag ccgctacagt 4560
caacagcaac tgatggaaac cagccatcgc catctgctgc acgcggaaga aggcacatgg 4620
ctgaatatcg acggtttcca tatggggatt ggtggcgacg actcctggag cccgtcagta 4680
tcggcggaat tccagctgag cgccggtcgc taccattacc agttggtctg gtgtcaaaaa 4740
taataataac cgggcagggg ggatctttgt gaaggaacct tacttctgtg gtgtgacata 4800
attggacaaa ctacctacag agatttaaag ctctaaggta aatataaaat ttttaagtgt 4860
ataatgtgtt aaactactga ttctaattgt ttgtgtattt tagattccaa cctatggaac 4920
tgatgaatgg gagcagtggt ggaatgccag atccagacat gataagatac attgatgagt 4980
ttggacaaac cacaactaga atgcagtgaa aaaaatgctt tatttgtgaa atttgtgatg 5040
ctattgcttt atttgtaacc attataagct gcaataaaca agttaacaac aacaattgca 5100
ttcattttat gtttcaggtt cagggggagg tgtgggaggt tttttaaagc aagtaaaacc 5160
tctacaaatg tggtatggct gattatgatc tgcggccgca gggcctcgtg atacgcctat 5220
ttttataggt taatgtcatg ataataatgg tttcttagac gtcaggtggc acttttcggg 5280
gaaatgtgcg cggaacccct atttgtttat ttttctaaat acattcaaat atgtatccgc 5340
tcatgagaca ataaccctga taaatgcttc aataatattg aaaaaggaag agtatgagta 5400
ttcaacattt ccgtgtcgcc cttattccct tttttgcggc attttgcctt cctgtttttg 5460
ctcacccaga aacgctggtg aaagtaaaag atgctgaaga tcagttgggt gcacgagtgg 5520
gttacatcga actggatctc aacagcggta agatccttga gagttttcgc cccgaagaac 5580
gttttccaat gatgagcact tttaaagttc tgctatgtgg cgcggtatta tcccgtattg 5640
acgccgggca agagcaactc ggtcgccgca tacactattc tcagaatgac ttggttgagt 5700
actcaccagt cacagaaaag catcttacgg atggcatgac agtaagagaa ttatgcagtg 5760
ctgccataac catgagtgat aacactgcgg ccaacttact tctgacaacg atcggaggac 5820
cgaaggagct aaccgctttt ttgcacaaca tgggggatca tgtaactcgc cttgatcgtt 5880
gggaaccgga gctgaatgaa gccataccaa acgacgagcg tgacaccacg atgcctgtag 5940
caatggcaac aacgttgcgc aaactattaa ctggcgaact acttactcta gcttcccggc 6000
aacaattaat agactggatg gaggcggata aagttgcagg accacttctg cgctcggccc 6060
ttccggctgg ctggtttatt gctgataaat ctggagccgg tgagcgtggg tctcgcggta 6120
tcattgcagc actggggcca gatggtaagc cctcccgtat cgtagttatc tacacgacgg 6180
ggagtcaggc aactatggat gaacgaaata gacagatcgc tgagataggt gcctcactga 6240
ttaagcattg gtaactgtca gaccaagttt actcatatat actttagatt gatttaaaac 6300
ttcattttta atttaaaagg atctaggtga agatcctttt tgataatctc atgaccaaaa 6360
tcccttaacg tgagttttcg ttccactgag cgtcagaccc cgtagaaaag atcaaaggat 6420
cttcttgaga tccttttttt ctgcgcgtaa tctgctgctt gcaaacaaaa aaaccaccgc 6480
taccagcggt ggtttgtttg ccggatcaag agctaccaac tctttttccg aaggtaactg 6540
gcttcagcag agcgcagata ccaaatactg tccttctagt gtagccgtag ttaggccacc 6600
acttcaagaa ctctgtagca ccgcctacat acctcgctct gctaatcctg ttaccagtgg 6660
ctgctgccag tggcgataag tcgtgtctta ccgggttgga ctcaagacga tagttaccgg 6720
ataaggcgca gcggtcgggc tgaacggggg gttcgtgcac acagcccagc ttggagcgaa 6780
cgacctacac cgaactgaga tacctacagc gtgagctatg agaaagcgcc acgcttcccg 6840
aagggagaaa ggcggacagg tatccggtaa gcggcagggt cggaacagga gagcgcacga 6900
gggagcttcc agggggaaac gcctggtatc tttatagtcc tgtcgggttt cgccacctct 6960
gacttgagcg tcgatttttg tgatgctcgt caggggggcg gagcctatgg aaaaacgcca 7020
gcaacgcggc ctttttacgg ttcctggcct tttgctggcc ttttgctcac atgttctttc 7080
ctgcgttatc ccctgattct gtggataacc gtattaccgc ctttgagtga gctgataccg 7140
ctcgccgcag ccgaacgacc gagcgcagcg agtcagtgag cgaggaagcg gaagagcgcc 7200

caatacgcaa accgcctctc cccgcgcgtt ggccgattca ttaatgcagc tggcacgaca 7260
ggtttcccga ctggaaagcg ggcagtgagc gcaacgcaat taatgtgagt tagctcactc 7320
attaggcacc ccaggcttta cactttatgc ttccggctcg tatgttgtgt ggaattgtga 7380
gcggataaca atttcacaca ggaaacagct atgaccatga ttacgccaag ctggcgcg 7438

<210> 6
<211> 45
<212> DNA
<213> Artificial Sequence

<220>
<223> Description of Artificial Sequence: primer C31-1

<400> 6
ataagaatgc ggccgcccga tatgacacaa ggggttgtga ccggg 45

<210> 7
<211> 40
<212> DNA
<213> Artificial Sequence

<220>
<223> Description of Artificial Sequence: primer C31-3

<400> 7
ataagaatgc ggccgcatcc gccgctacgt cttccgtgcc 40

<210> 8
<211> 24
<212> DNA
<213> Artificial Sequence

<220>

<223> Description of Artificial Sequence: primer C31-8

<400> 8
cccgttggca ggaagcactt ccgg 24

<210> 9
<211> 55
<212> DNA
<213> Artificial Sequence

<220>
<223> Description of Artificial Sequence: primer C31-9

<400> 9
ggatcctcga gccgcgggcg gccgcctacg ccgctacgtc ttccgtgccg tcctg 55

<210> 10
<211> 5711
<212> DNA
<213> Artificial Sequence

<220>
<223> Description of Artificial Sequence: vector
      pCMV-C31-Int(wt)

<400> 10
aaacagtccg atgtacgggc cagatatacg cgttgacatt gattattgac tagttattaa 60
tagtaatcaa ttacggggtc attagttcat agcccatata tggagttccg cgttacataa 120

cttacggtaa atggcccgcc tggctgaccg cccaacgacc cccgcccatt gacgtcaata 180
atgacgtatg ttcccatagt aacgccaata gggactttcc attgacgtca atgggtggac 240
tatttacggt aaactgccca cttggcagta catcaagtgt atcatatgcc aagtacgccc 300
cctattgacg tcaatgacgg taaatggccc gcctggcatt atgcccagta catgacctta 360
tgggactttc ctacttggca gtacatctac gtattagtca tcgctattac catggtgatg 420
cggttttggc agtacatcaa tgggcgtgga tagcggtttg actcacgggg atttccaagt 480
ctccacccca ttgacgtcaa tgggagtttg ttttggcacc aaaatcaacg ggactttcca 540
aaatgtcgta acaactccgc cccattgacg caaatgggcg gtaggcgtgt acggtgggag 600
gtctatataa gcagagctct ctggctaact agagaaccca ctgcttactg gcttatcgaa 660
attaatacga ctcactatag ggagacccaa gctgactcta gacttaatta agcgttgggg 720
tgagtactcc ctctcaaaag cgggcatgac ttctgcgcta agattgtcag tttccaaaaa 780
cgaggaggat ttgatattca cctggcccgc ggtgatgcct ttgagggtgg ccgcgtccat 840
ctggtcagaa aagacaatct ttttgttgtc aagcttgagg tgtggcaggc ttgagatctg 900
gccatacact tgagtgacat tgacatccac tttgcctttc tctccacagg tgtccactcc 960
cagggcggcc gcccgatatg acacaagggg ttgtgaccgg ggtggacacg tacgcgggtg 1020
cttacgaccg tcagtcgcgc gagcgcgaga attcgagcgc agcaagccca gcgacacagc 1080
gtagcgccaa cgaagacaag gcggccgacc ttcagcgcga agtcgagcgc gacgggggcc 1140
ggttcaggtt cgtcgggcat ttcagcgaag cgccgggcac gtcggcgttc gggacggcgg 1200
agcgcccgga gttcgaacgc atcctgaacg aatgccgcgc cgggcggctc aacatgatca 1260
ttgtctatga cgtgtcgcgc ttctcgcgcc tgaaggtcat ggacgcgatt ccgattgtct 1320
cggaattgct cgccctgggc gtgacgattg tttccactca ggaaggcgtc ttccggcagg 1380
gaaacgtcat ggacctgatt cacctgatta tgcggctcga cgcgtcgcac aaagaatctt 1440
cgctgaagtc ggcgaagatt ctcgacacga agaaccttca gcgcgaattg ggcgggtacg 1500
tcggcgggaa ggcgccttac ggcttcgagc ttgtttcgga gacgaaggag atcacgcgca 1560
acggccgaat ggtcaatgtc gtcatcaaca agcttgcgca ctcgaccact ccccttaccg 1620
gacccttcga gttcgagccc gacgtaatcc ggtggtggtg gcgtgagatc aagacgcaca 1680
aacaccttcc cttcaagccg ggcagtcaag ccgccattca cccgggcagc atcacggggc 1740
tttgtaagcg catggacgct gacgccgtgc cgacccgggg cgagacgatt gggaagaaga 1800
ccgcttcaag cgcctgggac ccggcaaccg ttatgcgaat ccttcgggac ccgcgtattg 1860
cgggcttcgc cgctgaggtg atctacaaga agaagccgga cggcacgccg accacgaaga 1920
ttgagggtta ccgcattcag cgcgacccga tcacgctccg gccggtcgag cttgattgcg 1980
gaccgatcat cgagcccgct gagtggtatg agcttcaggc gtggttggac ggcagggggc 2040
gcggcaaggg gctttcccgg gggcaagcca ttctgtccgc catggacaag ctgtactgcg 2100
agtgtggcgc cgtcatgact tcgaagcgcg gggaagaatc gatcaaggac tcttaccgct 2160
gccgtcgccg gaaggtggtc gacccgtccg cacctgggca gcacgaaggc acgtgcaacg 2220
tcagcatggc ggcactcgac aagttcgttg cggaacgcat cttcaacaag atcaggcacg 2280
ccgaaggcga cgaagagacg ttggcgcttc tgtgggaagc cgcccgacgc ttcggcaagc 2340
tcactgaggc gcctgagaag agcggcgaac gggcgaacct tgttgcggag cgcgccgacg 2400
ccctgaacgc ccttgaagag ctgtacgaag accgcgcggc aggcgcgtac gacggacccg 2460
ttggcaggaa gcacttccgg aagcaacagg cagcgctgac gctccggcag caaggggcgg 2520
aagagcggct tgccgaactt gaagccgccg aagccccgaa gcttcccctt gaccaatggt 2580
tccccgaaga cgccgacgct gacccgaccg gccctaagtc gtggtggggg cgcgcgtcag 2640
tagacgacaa gcgcgtgttc gtcgggctct tcgtagacaa gatcgttgtc acgaagtcga 2700
ctacgggcag gggcagggga acgcccatcg agaagcgcgc ttcgatcacg tgggcgaagc 2760
cgccgaccga cgacgacgaa gacgacgccc aggacggcac ggaagacgta gcggcgtagg 2820
cggcgcccgg gctcgagatc caggcgcgga tcaataaaag atcattattt tcaatagatc 2880
tgtgtgttgg ttttttgtgt gccttggggg agggggaggc cagaatgagg cgcggccaag 2940
ggggaggggg aggccagaat gaccttgggg gagggggagg ccagaatgac cttgggggag 3000
ggggaggcca gaatgaggcg cgcccccggg taccgagctc gaattcactg gccgtcgttt 3060
tacaacgtcg tgactgggaa aaccctggcg ttacccaact taatcgcctt gcagcacatc 3120
ccccttcgc cagctggcgt aatagcgaag aggcccgcac cgatcgccct tcccaacagt 3180
tgcgcagcct gaatggcgaa tggcgcctga tgcggtattt tctccttacg catctgtgcg 3240
gtatttcaca ccgcatatgg tgcactctca gtacaatctg ctctgatgcc gcatagttaa 3300
gccagccccg acacccgcca acacccgctg acgcgccctg acgggcttgt ctgctcccgg 3360
catccgctta cagacaagct gtgaccgtct ccgggagctg catgtgtcag aggttttcac 3420
cgtcatcacc gaaacgcgcg agacgaaagg gcctcgtgat acgcctattt ttataggtta 3480
atgtcatgat aataatggtt tcttagacgt caggtggcac ttttcgggga aatgtgcgcg 3540
gaacccctat ttgtttattt ttctaaatac attcaaatat gtatccgctc atgagacaat 3600
aaccctgata aatgcttcaa taatattgaa aaaggaagag tatgagtatt caacatttcc 3660
gtgtcgccct tattcccttt tttgcggcat tttgccttcc tgtttttgct cacccagaaa 3720
cgctggtgaa agtaaaagat gctgaagatc agttgggtgc acgagtgggt tacatcgaac 3780
tggatctcaa cagcggtaag atccttgaga gttttcgccc cgaagaacgt tttccaatga 3840
tgagcacttt taaagttctg ctatgtggcg cggtattatc ccgtattgac gccgggcaag 3900
agcaactcgg tcgccgcata cactattctc agaatgactt ggttgagtac tcaccagtca 3960
cagaaaagca tcttacggat ggcatgacag taagagaatt atgcagtgct gccataacca 4020
tgagtgataa cactgcggcc aacttacttc tgacaacgat cggaggaccg aaggagctaa 4080
ccgctttttt gcacaacatg ggggatcatg taactcgcct tgatcgttgg gaaccggagc 4140

tgaatgaagc cataccaaac gacgagcgtg acaccacgat gcctgtagca atggcaacaa 4200
cgttgcgcaa actattaact ggcgaactac ttactctagc ttcccggcaa caattaatag 4260
actggatgga ggcggataaa gttgcaggac cacttctgcg ctcggccctt ccggctggct 4320
ggtttattgc tgataaatct ggagccggtg agcgtgggtc tcgcggtatc attgcagcac 4380
tggggccaga tggtaagccc tcccgtatcg tagttatcta cacgacgggg agtcaggcaa 4440
ctatggatga acgaaataga cagatcgctg agataggtgc ctcactgatt aagcattggt 4500
aactgtcaga ccaagtttac tcatatatac tttagattga tttaaaactt catttttaat 4560
ttaaaaggat ctaggtgaag atcctttttg ataatctcat gaccaaaatc ccttaacgtg 4620
agttttcgtt ccactgagcg tcagaccccg tagaaaagat caaaggatct tcttgagatc 4680
ctttttttct gcgcgtaatc tgctgcttgc aaacaaaaaa accaccgcta ccagcggtgg 4740
tttgtttgcc ggatcaagag ctaccaactc tttttccgaa ggtaactggc ttcagcagag 4800
cgcagatacc aaatactgtc cttctagtgt agccgtagtt aggccaccac ttcaagaact 4860
ctgtagcacc gcctacatac ctcgctctgc taatcctgtt accagtggct gctgccagtg 4920
gcgataagtc gtgtcttacc gggttggact caagacgata gttaccggat aaggcgcagc 4980
ggtcgggctg aacggggggt tcgtgcacac agcccagctt ggagcgaacg acctacaccg 5040
aactgagata cctacagcgt gagctatgag aaagcgccac gcttcccgaa gggagaaagg 5100
cggacaggta tccggtaagc ggcagggtcg gaacaggaga gcgcacgagg gagcttccag 5160
ggggaaacgc ctggtatctt tatagtcctg tcgggtttcg ccacctctga cttgagcgtc 5220
gatttttgtg atgctcgtca ggggggcgga gcctatggaa aaacgccagc aacgcggcct 5280
ttttacggtt cctggccttt tgctggcctt ttgctcacat gttctttcct gcgttatccc 5340
ctgattctgt ggataaccgt attaccgcct ttgagtgagc tgataccgct cgccgcagcc 5400
gaacgaccga gcgcagcgag tcagtgagcg aggaagcgga agagcgccca atacgcaaac 5460
cgcctctccc cgcgcgttgg ccgattcatt aatgcagctg gcacgacagg tttcccgact 5520
ggaaagcggg cagtgagcgc aacgcaatta atgtgagtta gctcactcat taggcacccc 5580
aggctttaca ctttatgctt ccggctcgta tgttgtgtgg aattgtgagc ggataacaat 5640
ttcacacagg aaacagctat gaccatgatt acgccaagct agcccgggct agcttgcatg 5700
cctgcaggtt t 5711

<210> 11
<211> 69
<212> DNA
<213> Artificial Sequence

<220>
<223> Description of Artificial Sequence: primer C31-2-2

<400> 11
tagaattccg ctcgagagtc taaaccttcc tcttcttctt aggcgccgct acgtcttccg 60
tgccgtcct 69

<210> 12
<211> 5723
<212> DNA
<213> Artificial Sequence

<220>
<223> Description of Artificial Sequence: vector
pCMV-C31-Int(NLS)

<400> 12
cctgcaggtt taaacagtcc gatgtacggg ccagatatac gcgttgacat tgattattga 60
ctagttatta atagtaatca attacggggt cattagttca tagcccatat atggagttcc 120
gcgttacata acttacggta aatggcccgc ctggctgacc gcccaacgac ccccgcccat 180
tgacgtcaat aatgacgtat gttcccatag taacgccaat agggactttc cattgacgtc 240
aatgggtgga ctatttacgg taaactgccc acttggcagt acatcaagtg tatcatatgc 300
caagtacgcc ccctattgac gtcaatgacg gtaaatggcc cgcctggcat tatgcccagt 360
acatgacctt atgggacttt cctacttggc agtacatcta cgtattagtc atcgctatta 420
ccatggtgat gcggttttgg cagtacatca atgggcgtgg atagcggttt gactcacggg 480
gatttccaag tctccacccc attgacgtca atgggagttt gttttggcac caaaatcaac 540
gggactttcc aaaatgtcgt aacaactccg ccccattgac gcaaatgggc ggtaggcgtg 600
tacggtggga ggtctatata agcagagctc tctggctaac tagagaaccc actgcttact 660
ggcttatcga aattaatacg actcactata gggagaccca agctgactct agacttaatt 720
aagcgttggg gtgagtactc cctctcaaaa gcgggcatga cttctgcgct aagattgtca 780
gtttccaaaa acgaggagga tttgatattc acctggcccg cggtgatgcc tttgagggtg 840
gccgcgtcca tctggtcaga aaagacaatc tttttgttgt caagcttgag gtgtggcagg 900

cttgagatct ggccatacac ttgagtgaca ttgacatcca ctttgccttt ctctccacag 960
gtgtccactc ccagggcggc cgcccgatat gacacaaggg gttgtgaccg gggtggacac 1020
gtacgcgggt gcttacgacc gtcagtcgcg cgagcgcgag aattcgagcg cagcaagccc 1080
agcgacacag cgtagcgcca acgaagacaa ggcggccgac cttcagcgcg aagtcgagcg 1140
cgacgggggc cggttcaggt tcgtcggcca tttcagcgaa gcgccgggca cgtcggcgtt 1200
cgggacggcg gagcgcccgg agttcgaacg catcctgaac gaatgccgcg ccgggcggct 1260
caacatgatc attgtctatg acgtgtcgcg cttctcgcgc ctgaaggtca tggacgcgat 1320
tccgattgtc tcggaattgc tcgccctggg cgtgacgatt gtttccactc aggaaggcgt 1380
cttccggcag ggaaacgtca tggacctgat tcacctgatt atgcggctcg acgcgtcgca 1440
caaagaatct tcgctgaagt cggcgaagat tctcgacacg aagaaccttc agcgcgaatt 1500
gggcgggtac gtcggcggga aggcgcctta cggcttcgag cttgtttcgg agacgaagga 1560
gatcacgcgc aacggccgaa tggtcaatgt cgtcatcaac aagcttgcgc actcgaccac 1620
tccccttacc ggacccttcg agttcgagcc cgacgtaatc cggtggtggt ggcgtgagat 1680
caagacgcac aaacaccttc ccttcaagcc gggcagtcaa gccgccattc acccgggcag 1740
catcacgggg ctttgtaagc gcatggacgc tgacgccgtg ccgacccggg gcgagacgat 1800
tgggaagaag accgcttcaa gcgcctggga cccggcaacc gttatgcgaa tccttcggga 1860
cccgcgtatt gcgggcttcg ccgctgaggt gatctacaag aagaagccgg acggcacgcc 1920
gaccacgaag attgagggtt accgcattca gcgcgacccg atcacgctcc ggccggtcga 1980
gcttgattgc ggaccgatca tcgagcccgc tgagtggtat gagcttcagg cgtggttgga 2040
cggcaggggg cgcggcaagg ggctttcccg ggggcaagcc attctgtccg ccatggacaa 2100
gctgtactgc gagtgtggcg ccgtcatgac ttcgaagcgc ggggaagaat cgatcaagga 2160
ctcttaccgc tgccgtcgcc ggaaggtggt cgacccgtcc gcacctgggc agcacgaagg 2220
cacgtgcaac gtcagcatgg cggcactcga caagttcgtt gcggaacgca tcttcaacaa 2280
gatcaggcac gccgaaggcg acgaagagac gttggcgctt ctgtgggaag ccgcccgacg 2340
cttcggcaag ctcactgagg cgcctgagaa gagcggcgaa cgggcgaacc ttgttgcgga 2400
gcgcgccgac gccctgaacg cccttgaaga gctgtacgaa gaccgcgcgg caggcgcgta 2460
cgacggaccc gttggcagga agcacttccg gaagcaacag gcagcgctga cgctccggca 2520
gcaaggggcg gaagagcggc ttgccgaact tgaagccgcc gaagccccga agcttcccct 2580
tgaccaatgg ttccccgaag acgccgacgc tgacccgacc ggccctaagt cgtggtgggg 2640
gcgcgcgtca gtagacgaca agcgcgtgtt cgtcgggctc ttcgtagaca agatcgttgt 2700
cacgaagtcg actacgggca gggggcaggg aacgcccatc gagaagcgcg cttcgatcac 2760
gtgggcgaag ccgccgaccg acgacgacga agacgacgcc caggacggca cggaagacgt 2820
agcggcgcct aagaagaaga ggaaggttta gactctcgag atccaggcgc ggatcaataa 2880
aagatcatta ttttcaatag atctgtgtgt tggttttttg tgtgccttgg gggaggggga 2940
ggccagaatg aggcgcggcc aagggggagg gggaggccag aatgaccttg gggagggggg 3000
aggccagaat gaccttgggg gagggggagg ccagaatgag gcgcgccccc gggtaccgag 3060
ctcgaattca ctggccgtcg ttttacaacg tcgtgactgg gaaaaccctg gcgttaccca 3120
acttaatcgc cttgcagcac atcccccttt cgccagctgg cgtaatagcg aagaggcccg 3180
caccgatcgc ccttcccaac agttgcgcag cctgaatggc gaatggcgcc tgatgcggta 3240
ttttctcctt acgcatctgt gcggtatttc acaccgcata tggtgcactc tcagtacaat 3300
ctgctctgat gccgcatagt taagccagcc ccgacacccg ccaacacccg ctgacgcgcc 3360
ctgacgggct tgtctgctcc cggcatccgc ttacagacaa gctgtgaccg tctccgggag 3420
ctgcatgtgt cagaggtttt caccgtcatc accgaaacgc gcgagacgaa agggcctcgt 3480
gatacgccta tttttatagg ttaatgtcat gataataatg gtttcttaga cgtcaggtgg 3540
cacttttcgg ggaaatgtgc gcggaacccc tatttgttta tttttctaaa tacattcaaa 3600
tatgtatccg ctcatgagac aataaccctg ataaatgctt caataatatt gaaaaaggaa 3660
gagtatgagt attcaacatt tccgtgtcgc ccttattccc ttttttgcgg cattttgcct 3720
tcctgttttt gctcacccag aaacgctggt gaaagtaaaa gatgctgaag atcagttggg 3780
tgcacgagtg ggttacatcg aactggatct caacagcggt aagatccttg agagttttcg 3840
ccccgaagaa cgttttccaa tgatgagcac ttttaaagtt ctgctatgtg gcgcggtatt 3900
atcccgtatt gacgccgggc aagagcaact cggtcgccgc atacactatt ctcagaatga 3960
cttggttgag tactcaccag tcacagaaaa gcatcttacg gatggcatga cagtaagaga 4020
attatgcagt gctgccataa ccatgagtga taacactgcg gccaacttac ttctgacaac 4080
gatcggagga ccgaaggagc taaccgcttt tttgcacaac atgggggatc atgtaactcg 4140
ccttgatcgt tgggaaccgg agctgaatga agccatacca aacgacgagc gtgacaccac 4200
gatgcctgta gcaatggcaa caacgttgcg caaactatta actggcgaac tacttactct 4260
agcttcccgg caacaattaa tagactggat ggaggcggat aaagttgcag gaccacttct 4320
gcgctcggcc cttccggctg gctggtttat tgctgataaa tctggagccg gtgagcgtgg 4380
gtctcgcggt atcattgcag cactggggcc agatggtaag ccctcccgta tcgtagttat 4440
ctacacgacg gggagtcagg caactatgga tgaacgaaat agacagatcg ctgagatagg 4500
tgcctcactg attaagcatt ggtaactgtc agaccaagtt tactcatata tactttagat 4560
tgatttaaaa cttcattttt aatttaaaag gatctaggtg aagatccttt ttgataatct 4620
catgaccaaa atcccttaac gtgagttttc gttccactga gcgtcagacc ccgtagaaaa 4680
gatcaaagga tcttcttgag atcctttttt tctgcgcgta atctgctgct tgcaaacaaa 4740
aaaaccaccg ctaccagcgg tggtttgttt gccggatcaa gagctaccaa ctctttttcc 4800
gaaggtaact ggcttcagca gagcgcagat accaaatact gtccttctag tgtagccgta 4860
gttaggccac cacttcaaga actctgtagc accgcctaca tacctcgctc tgctaatcct 4920

gttaccagtg gctgctgcca gtggcgataa gtcgtgtctt accgggttgg actcaagacg 4980
atagttaccg gataaggcgc agcggtcggg ctgaacgggg ggttcgtgca cacagcccag 5040
cttggagcga acgacctaca ccgaactgag atacctacag cgtgagctat gagaaagcgc 5100
cacgcttccc gaagggagaa aggcggacag gtatccggta agcggcaggg tcggaacagg 5160
agagcgcacg agggagcttc cagggggaaa cgcctggtat ctttatagtc ctgtcgggtt 5220
tcgccacctc tgacttgagc gtcgattttt gtgatgctcg tcaggggggc ggagcctatg 5280
gaaaaacgcc agcaacgcgg cctttttacg gttcctggcc ttttgctggc cttttgctca 5340
catgttcttt cctgcgttat cccctgattc tgtggataac cgtattaccg cctttgagtg 5400
agctgatacc gctcgccgca gccgaacgac cgagcgcagc gagtcagtga gcgaggaagc 5460
ggaagagcgc ccaatacgca aaccgcctct ccccgcgcgt tggccgattc attaatgcag 5520
ctggcacgac aggtttcccg actggaaagc gggcagtgag cgcaacgcaa ttaatgtgag 5580
ttagctcact cattaggcac cccaggcttt acactttatg cttccggctc gtatgttgtg 5640
tggaattgtg agcggataac aatttcacac aggaaacagc tatgaccatg attacgccaa 5700
gctagcccgg gctagcttgc atg 5723

<210> 13
<211> 4960
<212> DNA
<213> Artificial Sequence

<220>
<223> Description of Artificial Sequence: vector
      pCMV-Cre

<400> 13
aaacagtccg atgtacgggc cagatatacg cgttgacatt gattattgac tagttattaa 60
tagtaatcaa ttacggggtc attagttcat agcccatata tggagttccg cgttacataa 120
cttacggtaa atggcccgcc tggctgaccg cccaacgacc cccgcccatt gacgtcaata 180
atgacgtatg ttcccatagt aacgccaata gggactttcc attgacgtca atgggtggac 240
tatttacggt aaactgccca cttggcagta catcaagtgt atcatatgcc aagtacgccc 300
cctattgacg tcaatgacgg taaatggccc gcctggcatt atgcccagta catgacctta 360
tgggactttc ctacttggca gtacatctac gtattagtca tcgctattac catggtgatg 420
cggttttggc agtacatcaa tgggcgtgga tagcggtttg actcacgggg atttccaagt 480
ctccacccca ttgacgtcaa tgggagtttg ttttggcacc aaaatcaacg ggactttcca 540
aaatgtcgta acaactccgc cccattgacg caaatgggcg gtaggcgtgt acggtgggag 600
gtctatataa gcagagctct ctggctaact agagaaccca ctgcttactg gcttatcgaa 660
attaatacga ctcactatag ggagacccaa gctgactcta gacttaatta agcgttgggg 720
tgagtactcc ctctcaaaag cgggcatgac ttctgcgcta agattgtcag tttccaaaaa 780
cgaggaggat ttgatattca cctggcccgc ggtgatgcct ttgagggtgg ccgcgtccat 840
ctggtcagaa aagacaatct ttttgttgtc aagcttgagg tgtggcaggc ttgagatctg 900
gccatacact tgagtgacat tgacatccac tttgcctttc tctccacagg tgtccactcc 960
cagggcggcc tcgaccatgc ccaagaagaa gaggaaggtg tccaatttac tgaccgtaca 1020
ccaaaatttg cctgcattac cggtcgatgc aacgagtgat gaggttcgca agaacctgat 1080
ggacatgttc agggatcgcc aggcgttttc tgagcatacc tggaaaatgc ttctgtccgt 1140
ttgccggtcg tgggcggcat ggtgcaagtt gaataaccgg aaatggtttc ccgcagaacc 1200
tgaagatgtt cgcgattatc ttctatatct tcaggcgcgc ggtctggcag taaaaactat 1260
ccagcaacat ttgggccagc taaacatgct tcatcgtcgg tccgggctgc cacgaccaag 1320
tgacagcaat gctgtttcac tggttatgcg gcggatccga aaagaaaacg ttgatgccgg 1380
tgaacgtgca aaacaggctc tagcgttcga acgcactgat ttcgaccagg ttcgttcact 1440
catggaaaat agcgatcgct gccaggatat acgtaatctg gcatttctgg ggattgctta 1500
taacaccctg ttacgtatag ccgaaattgc caggatcagg gttaaagata tctcacgtac 1560
tgacggtggg agaatgttaa tccatattgg cagaacgaaa acgctggtta gcaccgcagg 1620
tgtagagaag gcacttagcc tgggggtaac taaactggtc gagcgatgga tttccgtctc 1680
tggtgtagct gatgatccga ataactacct gttttgccgg gtcagaaaaa atggtgttgc 1740
cgcgccatct gccaccagcc agctatcaac tcgcgccctg gaagggattt ttgaagcaac 1800
tcatcgattg atttacggcg ctaaggatga ctctggtcag agatacctgg cctggtctgg 1860
acacagtgcc cgtgtcggag ccgcgcgaga tatggcccgc gctggagttt caataccgga 1920
gatcatgcaa gctggtggct ggaccaatgt aaatattgtc atgaactata tccgtaacct 1980
ggatagtgaa acaggggcaa tggtgcgcct gctggaagat ggcgattagc cattaacgcg 2040
taaatgattg cagatccact agttctaggg ccgcgtcgac ctcgagatcc aggcgcggat 2100
caataaaaga tcattatttt caatagatct gtgtgttggt tttttgtgtg ccttggggga 2160
gggggaggcc agaatgaggc gcggccaagg gggaggggga ggccagaatg accttggggg 2220
agggggaggc cagaatgacc ttgggggagg gggaggccag aatgaggcgc gcccccgggt 2280
accgagctcg aattcactgg ccgtcgtttt acaacgtcgt gactgggaaa accctggcgt 2340
tacccaactt aatcgccttg cagcacatcc ccctttcgcc agctggcgta atagcgaaga 2400
ggcccgcacc gatcgccctt cccaacagtt gcgcagcctg aatggcgaat ggcgcctgat 2460

gcggtatttt ctccttacgc atctgtgcgg tatttcacac cgcatatggt gcactctcag 2520

tacaatctgc tctgatgccg catagttaag ccagccccga cacccgccaa cacccgctga 2580
cgcgccctga cgggcttgtc tgctcccggc atccgcttac agacaagctg tgaccgtctc 2640
cgggagctgc atgtgtcaga ggttttcacc gtcatcaccg aaacgcgcga gacgaaaggg 2700
cctcgtgata cgcctatttt tataggttaa tgtcatgata ataatggttt cttagacgtc 2760
aggtggcact tttcggggaa atgtgcgcgg aacccctatt tgtttatttt tctaaataca 2820
ttcaaatatg tatccgctca tgagacaata accctgataa atgcttcaat aatattgaaa 2880
aaggaagagt atgagtattc aacatttccg tgtcgccctt attccctttt ttgcggcatt 2940
ttgccttcct gtttttgctc acccagaaac gctggtgaaa gtaaaagatg ctgaagatca 3000
gttgggtgca cgagtgggtt acatcgaact ggatctcaac agcggtaaga tccttgagag 3060
ttttcgcccc gaagaacgtt ttccaatgat gagcactttt aaagttctgc tatgtggcgc 3120
ggtattatcc cgtattgacg ccgggcaaga gcaactcggt cgccgcatac actattctca 3180
gaatgacttg gttgagtact caccagtcac agaaaagcat cttacggatg gcatgacagt 3240
aagagaatta tgcagtgctg ccataaccat gagtgataac actgcggcca acttacttct 3300
gacaacgatc ggaggaccga aggagctaac cgcttttttg cacaacatgg gggatcatgt 3360
aactcgcctt gatcgttggg aaccggagct gaatgaagcc ataccaaacg acgagcgtga 3420
caccacgatg cctgtagcaa tggcaacaac gttgcgcaaa ctattaactg gcgaactact 3480
tactctagct tcccggcaac aattaataga ctggatggag gcggataaag ttgcaggacc 3540
acttctgcgc tcggcccttc cggctggctg gtttattgct gataaatctg gagccggtga 3600
gcgtgggtct cgcggtatca ttgcagcact ggggccagat ggtaagccct cccgtatcgt 3660
agttatctac acgacgggga gtcaggcaac tatggatgaa cgaaatagac agatcgctga 3720
gataggtgcc tcactgatta agcattggta actgtcagac caagtttact catatatact 3780
ttagattgat ttaaaacttc atttttaatt taaaaggatc taggtgaaga tcctttttga 3840
taatctcatg accaaaatcc cttaacgtga gttttcgttc cactgagcgt cagaccccgt 3900
agaaaagatc aaaggatctt cttgagatcc tttttttctg cgcgtaatct gctgcttgca 3960
aacaaaaaaa ccaccgctac cagcggtggt ttgtttgccg gatcaagagc taccaactct 4020
ttttccgaag gtaactggct tcagcagagc gcagatacca aatactgtcc ttctagtgta 4080
gccgtagtta ggccaccact tcaagaactc tgtagcaccg cctacatacc tcgctctgct 4140
aatcctgtta ccagtggctg ctgccagtgg cgataagtcg tgtcttaccg ggttggactc 4200
aagacgatag ttaccggata aggcgcagcg gtcgggctga acggggggtt cgtgcacaca 4260
gcccagcttg gagcgaacga cctacaccga actgagatac ctacagcgtg agctatgaga 4320
aagcgccacg cttcccgaag ggagaaaggc ggacaggtat ccggtaagcg gcagggtcgg 4380
aacaggagag cgcacgaggg agcttccagg gggaaacgcc tggtatcttt atagtcctgt 4440
cgggtttcgc cacctctgac ttgagcgtcg atttttgtga tgctcgtcag gggggcggag 4500
cctatggaaa aacgccagca acgcggcctt tttacggttc ctggcctttt gctggccttt 4560
tgctcacatg ttctttcctg cgttatcccc tgattctgtg gataaccgta ttaccgcctt 4620
tgagtgagct gataccgctc gccgcagccg aacgaccgag cgcagcgagt cagtgagcga 4680
ggaagcggaa gagcgcccaa tacgcaaacc gcctctcccc gcgcgttggc cgattcatta 4740
atgcagctgg cacgacaggt ttcccgactg gaaagcgggc agtgagcgca acgcaattaa 4800
tgtgagttag ctcactcatt aggcacccca ggctttacac tttatgcttc cggctcgtat 4860
gttgtgtgga attgtgagcg gataacaatt tcacacagga aacagctatg accatgatta 4920
cgccaagcta gcccgggcta gcttgcatgc ctgcaggttt 4960

<210> 14
<211> 3858
<212> DNA
<213> Artificial Sequence

<220>
<223> Description of Artificial Sequence: vector pRK50

<400> 14
aaacagtccg atgtacgggc cagatatacg cgttgacatt gattattgac tagttattaa 60
tagtaatcaa ttacggggtc attagttcat agcccatata tggagttccg cgttacataa 120
cttacggtaa atggcccgcc tggctgaccg cccaacgacc cccgcccatt gacgtcaata 180
atgacgtatg ttcccatagt aacgccaata gggactttcc attgacgtca atgggtggac 240
tatttacggt aaactgccca cttggcagta catcaagtgt atcatatgcc aagtacgccc 300
cctattgacg tcaatgacgg taaatggccc gcctggcatt atgcccagta catgacctta 360
tgggactttc ctacttggca gtacatctac gtattagtca tcgctattac catggtgatg 420
cggttttggc agtacatcaa tgggcgtgga tagcggtttg actcacgggg atttccaagt 480
ctccacccca ttgacgtcaa tgggagtttg ttttggcacc aaaatcaacg ggactttcca 540
aaatgtcgta acaactccgc cccattgacg caaatgggcg gtaggcgtgt acggtgggag 600
gtctatataa gcagagctct ctggctaact agagaaccca ctgcttactg gcttatcgaa 660
attaatacga ctcactatag ggagacccaa gctgactcta gacttaatta agcgttgggg 720
tgagtactcc ctctcaaaag cgggcatgac ttctgcgcta agattgtcag tttccaaaaa 780

cgaggaggat ttgatattca cctggcccgc ggtgatgcct ttgagggtgg ccgcgtccat 840
ctggtcagaa aagacaatct ttttgttgtc aagcttgagg tgtggcaggc ttgagatctg 900
gccatacact tgagtgacat tgacatccac tttgcctttc tctccacagg tgtccactcc 960
cagggcggcc gcgtcgacct cgagatccag gcgcggatca ataaaagatc attattttca 1020
atagatctgt gtgttggttt tttgtgtgcc ttgggggagg gggaggccag aatgaggcgc 1080
ggccaagggg gagggggagg ccagaatgac cttgggggag ggggaggcca gaatgacctt 1140
gggggagggg gaggccagaa tgaggcgcgc ccccgggtac cgagctcgaa ttcactggcc 1200
gtcgttttac aacgtcgtga ctgggaaaac cctggcgtta cccaacttaa tcgccttgca 1260
gcacatcccc ctttcgccag ctggcgtaat agcgaagagg cccgcaccga tcgcccttcc 1320
caacagttgc gcagcctgaa tggcgaatgg cgcctgatgc ggtattttct ccttacgcat 1380
ctgtgcggta tttcacaccg catatggtgc actctcagta caatctgctc tgatgccgca 1440
tagttaagcc agccccgaca cccgccaaca cccgctgacg cgccctgacg ggcttgtctg 1500
ctcccggcat ccgcttacag acaagctgtg accgtctccg ggagctgcat gtgtcagagg 1560
ttttcaccgt catcaccgaa acgcgcgaga cgaaagggcc tcgtgatacg cctattttta 1620
taggttaatg tcatgataat aatggtttct tagacgtcag gtggcacttt tcggggaaat 1680
gtgcgcggaa cccctatttg tttatttttc taaatacatt caaatatgta tccgctcatg 1740
agacaataac cctgataaat gcttcaataa tattgaaaaa ggaagagtat gagtattcaa 1800
catttccgtg tcgcccttat tccctttttt gcggcatttt gccttcctgt ttttgctcac 1860
ccagaaacgc tggtgaaagt aaaagatgct gaagatcagt tgggtgcacg agtgggttac 1920
atcgaactgg atctcaacag cggtaagatc cttgagagtt ttcgccccga agaacgtttt 1980
ccaatgatga gcacttttaa agttctgcta tgtggcgcgg tattatcccg tattgacgcc 2040
gggcaagagc aactcggtcg ccgcatacac tattctcaga atgacttggt tgagtactca 2100
ccagtcacag aaaagcatct tacggatggc atgacagtaa gagaattatg cagtgctgcc 2160
ataaccatga gtgataacac tgcggccaac ttacttctga caacgatcgg aggaccgaag 2220
gagctaaccg cttttttgca caacatgggg gatcatgtaa ctcgccttga tcgttgggaa 2280
ccggagctga atgaagccat accaaacgac gagcgtgaca ccacgatgcc tgtagcaatg 2340
gcaacaacgt tgcgcaaact attaactggc gaactactta ctctagcttc ccggcaacaa 2400
ttaatagact ggatggaggc ggataaagtt gcaggaccac ttctgcgctc ggcccttccg 2460
gctggctggt ttattgctga taaatctgga gccggtgagc gtgggtctcg cggtatcatt 2520
gcagcactgg ggccagatgg taagccctcc cgtatcgtag ttatctacac gacggggagt 2580
caggcaacta tggatgaacg aaatagacag atcgctgaga taggtgcctc actgattaag 2640
cattggtaac tgtcagacca agtttactca tatatacttt agattgattt aaaacttcat 2700
ttttaattta aaaggatcta ggtgaagatc ctttttgata atctcatgac caaaatccct 2760
taacgtgagt tttcgttcca ctgagcgtca gaccccgtag aaaagatcaa aggatcttct 2820
tgagatcctt tttttctgcg cgtaatctgc tgcttgcaaa caaaaaaacc accgctacca 2880
gcggtggttt gtttgccgga tcaagagcta ccaactcttt ttccgaaggt aactggcttc 2940
agcagagcgc agataccaaa tactgtcctt ctagtgtagc cgtagttagg ccaccacttc 3000
aagaactctg tagcaccgcc tacatacctc gctctgctaa tcctgttacc agtggctgct 3060
gccagtggcg ataagtcgtg tcttaccggg ttggactcaa gacgatagtt accggataag 3120
gcgcagcggt cgggctgaac ggggggttcg tgcacacagc ccagcttgga gcgaacgacc 3180
tacaccgaac tgagatacct acagcgtgag ctatgagaaa gcgccacgct tcccgaaggg 3240
agaaaggcgg acaggtatcc ggtaagcggc agggtcggaa caggagagcg cacgagggag 3300
cttccagggg gaaacgcctg gtatctttat agtcctgtcg ggtttcgcca cctctgactt 3360
gagcgtcgat ttttgtgatg ctcgtcaggg gggcggagcc tatggaaaaa cgccagcaac 3420
gcggcctttt tacggttcct ggccttttgc tggccttttg ctcacatgtt ctttcctgcg 3480
ttatcccctg attctgtgga taaccgtatt accgcctttg agtgagctga taccgctcgc 3540
cgcagccgaa cgaccgagcg cagcgagtca gtgagcgagg aagcggaaga gcgcccaata 3600
cgcaaaccgc ctctccccgc gcgttggccg attcattaat gcagctggca cgacaggttt 3660
cccgactgga aagcgggcag tgagcgcaac gcaattaatg tgagttagct cactcattag 3720
gcaccccagg ctttacactt tatgcttccg gctcgtatgt tgtgtggaat tgtgagcgga 3780
taacaatttc acacaggaaa cagctatgac catgattacg ccaagctagc ccgggctagc 3840
ttgcatgcct gcaggttt 3858

<210> 15
<211> 6257
<212> DNA
<213> Artificial Sequence

<220>
<223> Description of Artificial Sequence: vector
      pRK64(deltaCre)

<400> 15
cgtcatcacc gaaacgcgcg aggcagctgt ggaatgtgtg tcagttaggg tgtggaaagt 60
ccccaggctc cccagcaggc agaagtatgc aaagcatgca tctcaattag tcagcaacca 120
ggctccccag caggcagaag tgtgcaaagc atgcatctca attagtcagc aaccatagtc 180

ccgcccctaa ctccgcccat cccgccccta actccgccca gttccgccca ttctccgccc 240
catggctgac taattttttt tatttatgca gaggccgagg ccgcctcggc ctaggaacag 300
tcgacgacac tgcagagacc tacttcacta acaaccggta cagttcgtgg accagatggg 360
tgaggtggag tacgcgcccg ggagcccaag ggcacgccc tggcacccgc accgcggctt 420
cgagaccgtc acgaatagat ccataacttc gtatagcata cattatacga agttataccg 480
ggccaccatg gtcgcgagta gcttggcact ggccgtcgtt ttacaacgtc gtgactggga 540
aaaccctggc gttacccaac ttaatcgcct tgcagcacat ccccctttcg ccagctggcg 600
taatagcgaa gaggcccgca ccgatcgccc ttcccaacag ttgcgcagcc tgaatggcga 660
atggcgcttt gcctggtttc cggcaccaga agcggtgccg gaaagctggc tggagtgcga 720
tcttcctgag gccgatactg tcgtcgtccc ctcaaactgg cagatgcacg gttacgatgc 780
gcccatctac accaacgtaa cctatcccat tacggtcaat ccgccgtttg ttcccacgga 840
gaatccgacg ggttgttact cgctcacatt taatgttgat gaaagctggc tacaggaagg 900
ccagacgcga attatttttg atggcgttaa ctcggcgttt catctgtggt gcaacgggcg 960
ctgggtcggt tacggccagg acagtcgttt gccgtctgaa tttgacctga gcgcattttt 1020
acgcgccgga gaaaaccgcc tcgcggtgat ggtgctgcgt tggagtgacg gcagttatct 1080
ggaagatcag gatatgtggc ggatgagcgg cattttccgt gacgtctcgt tgctgcataa 1140
accgactaca caaatcagcg atttccatgt tgccactcgc tttaatgatg atttcagccg 1200
cgctgtactg gaggctgaag ttcagatgtg cggcgagttg cgtgactacc tacgggtaac 1260
agtttcttta tggcagggtg aaacgcaggt cgccagcggc accgcgcctt tcggcggtga 1320
aattatcgat gagcgtggtg gttatgccga tcgcgtcaca ctacgtctga acgtcgaaaa 1380
cccgaaactg tggagcgccg aaatcccgaa tctctatcgt gcggtggttg aactgcacac 1440
cgccgacggc acgctgattg aagcagaagc ctgcgatgtc ggtttccgcg aggtgcggat 1500
tgaaaatggt ctgctgctgc tgaacggcaa gccgttgctg attcgaggcg ttaaccgtca 1560
cgagcatcat cctctgcatg gtcaggtcat ggatgagcag acgatggtgc aggatatcct 1620
gctgatgaag cagaacaact ttaacgccgt gcgctgttcg cattatccga accatccgct 1680
gtggtacacg ctgtgcgacc gctacggcct gtatgtggtg gatgaagcca atattgaaac 1740
ccacggcatg gtgccaatga atcgtctgac cgatgatccg cgctggctac cggcgatgag 1800
cgaacgcgta acgcgaatgg tgcagcgcga tcgtaatcac ccgagtgtga tcatctggtc 1860
gctggggaat gaatcaggcc acggcgctaa tcacgacgcg ctgtatcgct ggatcaaatc 1920
tgtcgatcct tcccgcccgg tgcagtatga aggcggcgga gccgacacca cggccaccga 1980
tattatttgc ccgatgtacg cgcgcgtgga tgaagaccag cccttcccgg ctgtgccgaa 2040
atggtccatc aaaaaatggc tttcgctacc tggagagacg cgcccgctga tcctttgcga 2100
atacgcccac gcgatgggta acagtcttgg cggtttcgct aaatactggc aggcgtttcg 2160
tcagtatccc cgtttacagg gcggcttcgt ctgggactgg gtggatcagt cgctgattaa 2220
atatgatgaa aacggcaacc cgtggtcggc ttacggcggt gattttggcg atacgccgaa 2280
cgatcgccag ttctgtatga acggtctggt ctttgccgac cgcacgccgc atccagcgct 2340
gacggaagca aaacaccagc agcagttttt ccagttccgt ttatccgggc aaaccatcga 2400
agtgaccagc gaatacctgt tccgtcatag cgataacgag ctcctgcact ggatggtggc 2460
gctggatggt aagccgctgg caagcggtga agtgcctctg gatgtcgctc cacaaggtaa 2520
acagttgatt gaactgcctg aactaccgca gccggagagc gccgggcaac tctggctcac 2580
agtacgcgta gtgcaaccga acgcgaccgc atggtcagaa gccgggcaca tcagcgcctg 2640
gcagcagtgg cgtctggcgg aaaacctcag tgtgacgctc cccgccgcgt cccacgccat 2700
cccgcatctg accaccagcg aaatggattt ttgcatcgag ctgggtaata agcgttggca 2760
atttaaccgc cagtcaggct ttctttcaca gatgtggatt ggcgataaaa aacaactgct 2820
gacgccgctg cgcgatcagt tcacccgtgc accgctggat aacgacattg gcgtaagtga 2880
agcgacccgc attgacccta acgcctgggt cgaacgctgg aaggcggcgg gccattacca 2940
ggccgaagca gcgttgttgc agtgcacggc agatacactt gctgatgcgg tgctgattac 3000
gaccgctcac gcgtggcagc atcaggggaa aaccttattt atcagccgga aaacctaccg 3060
gattgatggt agtggtcaaa tggcgattac cgttgatgtt gaagtggcga gcgatacacc 3120
gcatccggcg cggattggcc tgaactgcca gctggcgcag gtagcagagc gggtaaactg 3180
gctcggatta gggccgcaag aaaactatcc cgaccgcctt actgccgcct gttttgaccg 3240
ctgggatctg ccattgtcag acatgtatac cccgtacgtc ttcccgagcg aaaacggtct 3300
gcgctgcggg acgcgcgaat tgaattatgg cccacaccag tggcgcggcg acttccagtt 3360
caacatcagc cgctacagtc aacagcaact gatggaaacc agccatcgcc atctgctgca 3420
cgcggaagaa ggcacatggc tgaatatcga cggtttccat atggggattg gtggcgacga 3480
ctcctggagc ccgtcagtat cggcggaatt ccagctgagc gccggtcgct accattacca 3540
gttggtctgg tgtcaaaaat aataataacc gggcaggggg gatctttgtg aaggaacctt 3600
acttctgtgg tgtgacataa ttggacaaac tacctacaga gatttaaagc tctaaggtaa 3660
atataaaatt tttaagtgta taatgtgtta aactactgat tctaattgtt tgtgtatttt 3720
agattccaac ctatggaact gatgaatggg agcagtggtg gaatgccaga tccagacatg 3780
ataagataca ttgatgagtt tggacaaacc acaactagaa tgcagtgaaa aaaatgcttt 3840
atttgtgaaa tttgtgatgc tattgcttta tttgtaacca ttataagctg caataaacaa 3900
gttaacaaca acaattgcat tcattttatg tttcaggttc agggggaggt gtgggaggtt 3960
ttttaaagca agtaaaacct ctacaaatgt ggtatggctg attatgatct gcggccgcag 4020
ggcctcgtga tacgcctatt tttataggtt aatgtcatga taataatggt ttcttagacg 4080
tcaggtggca cttttcgggg aaatgtgcgc ggaaccccta tttgtttatt tttctaaata 4140
cattcaaata tgtatccgct catgagacaa taaccctgat aaatgcttca ataatattga 4200

aaaaggaaga gtatgagtat tcaacatttc cgtgtcgccc ttattccctt ttttgcggca 4260
ttttgccttc ctgtttttgc tcacccagaa acgctggtga aagtaaaaga tgctgaagat 4320
cagttgggtg cacgagtggg ttacatcgaa ctggatctca acagcggtaa gatccttgag 4380
agttttcgcc ccgaagaacg ttttccaatg atgagcactt ttaaagttct gctatgtggc 4440
gcggtattat cccgtattga cgccgggcaa gagcaactcg gtcgccgcat acactattct 4500
cagaatgact tggttgagta ctcaccagtc acagaaaagc atcttacgga tggcatgaca 4560
gtaagagaat tatgcagtgc tgccataacc atgagtgata acactgcggc caacttactt 4620
ctgacaacga tcggaggacc gaaggagcta accgcttttt tgcacaacat gggggatcat 4680
gtaactcgcc ttgatcgttg ggaaccggag ctgaatgaag ccataccaaa cgacgagcgt 4740
gacaccacga tgcctgtagc aatggcaaca acgttgcgca aactattaac tggcgaacta 4800
cttactctag cttcccggca acaattaata gactggatgg aggcggataa agttgcagga 4860
ccacttctgc gctcggccct tccggctggc tggtttattg ctgataaatc tggagccggt 4920
gagcgtgggt ctcgcggtat cattgcagca ctggggccag atggtaagcc ctcccgtatc 4980
gtagttatct acacgacggg gagtcaggca actatggatg aacgaaatag acagatcgct 5040
gagataggtg cctcactgat taagcattgg taactgtcag accaagttta ctcatatata 5100
ctttagattg atttaaaact tcatttttaa tttaaaagga tctaggtgaa gatccttttt 5160
gataatctca tgaccaaaat cccttaacgt gagttttcgt tccactgagc gtcagacccc 5220
gtagaaaaga tcaaaggatc ttcttgagat cctttttttc tgcgcgtaat ctgctgcttg 5280
caaacaaaaa aaccaccgct accagcggtg gtttgtttgc cggatcaaga gctaccaact 5340
ctttttccga aggtaactgg cttcagcaga gcgcagatac caaatactgt ccttctagtg 5400
tagccgtagt taggccacca cttcaagaac tctgtagcac cgcctacata cctcgctctg 5460
ctaatcctgt taccagtggc tgctgccagt ggcgataagt cgtgtcttac cgggttggac 5520
tcaagacgat agttaccgga taaggcgcag cggtcgggct gaacgggggg ttcgtgcaca 5580
cagcccagct tggagcgaac gacctacacc gaactgagat acctacagcg tgagctatga 5640
gaaagcgcca cgcttcccga agggagaaag gcggacaggt atccggtaag cggcagggtc 5700
ggaacaggag agcgcacgag ggagcttcca gggggaaacg cctggtatct ttatagtcct 5760
gtcgggtttc gccacctctg acttgagcgt cgatttttgt gatgctcgtc aggggggcgg 5820
agcctatgga aaaacgccag caacgcggcc tttttacggt tcctggcctt ttgctggcct 5880
tttgctcaca tgttctttcc tgcgttatcc cctgattctg tggataaccg tattaccgcc 5940
tttgagtgag ctgataccgc tcgccgcagc cgaacgaccg agcgcagcga gtcagtgagc 6000
gaggaagcgg aagagcgccc aatacgcaaa ccgcctctcc ccgcgcgttg gccgattcat 6060
taatgcagct ggcacgacag gtttcccgac tggaaagcgg gcagtgagcg caacgcaatt 6120
aatgtgagtt agctcactca ttaggcaccc caggctttac actttatgct tccggctcgt 6180
atgttgtgtg gaattgtgag cggataacaa tttcacacag gaaacagcta tgaccatgat 6240
tacgccaagc tggcgcg 6257

<210> 16
<211> 6252
<212> DNA
<213> Artificial Sequence

<220>
<223> Description of Artificial Sequence: vector
      pRK64(deltaInt)

<400> 16
cgtcatcacc gaaacgcgcg aggcagctgt ggaatgtgtg tcagttaggg tgtggaaagt 60
ccccaggctc cccagcaggc agaagtatgc aaagcatgca tctcaattag tcagcaacca 120
ggctccccag caggcagaag tgtgcaaagc atgcatctca attagtcagc aaccatagtc 180
ccgcccctaa ctccgcccat cccgccccta actccgccca gttccgccca ttctccgccc 240
catggctgac taattttttt tatttatgca gaggccgagg ccgcctcggc ctaggaacag 300
tcgacgacac tgcagagacc tacttcacta acaaccggta cagttcgtgg accagatggg 360
tgaggtggag tacgcgcccg gggagcccaa aggttacccc agttggggca ctactcccga 420
aaaccgcttc tggatccata acttcgtata gcatacatta tacgaagtta taccggccca 480
ccatggtcgc gagtagcttg gcactggccg tcgttttaca acgtcgtgac tgggaaaacc 540
ctggcgttac ccaacttaat cgccttgcag cacatccccc tttcgccagc tggcgtaata 600
gcgaagaggc ccgcaccgat cgcccttccc aacagttgcg cagcctgaat ggcgaatggc 660
gctttgcctg gtttccggca ccagaagcgg tgccggaaag ctggctggag tgcgatcttc 720
ctgaggccga tactgtcgtc gtcccctcaa actggcagat gcacggttac gatgcgccca 780
tctacaccaa cgtaacctat cccattacgg tcaatccgcc gtttgttccc acggagaatc 840
cgacgggttg ttactcgctc acatttaatg ttgatgaaag ctggctacag gaaggccaga 900
cgcgaattat ttttgatggc gttaactcgg cgtttcatct gtggtgcaac gggcgctggg 960
tcggttacgg ccaggacagt cgtttgccgt ctgaatttga cctgagcgca tttttacgcg 1020
ccggagaaaa ccgcctcgcg gtgatggtgc tgcgttggag tgacggcagt tatctggaag 1080
atcaggatat gtggcggatg agcggcattt tccgtgacgt ctcgttgctg cataaaccga 1140
ctacacaaat cagcgatttc catgttgcca ctcgctttaa tgatgatttc agccgcgctg 1200

tactggaggc tgaagttcag atgtgcggcg agttgcgtga ctacctacgg gtaacagttt 1260
ctttatggca gggtgaaacg caggtcgcca gcggcaccgc gcctttcggc ggtgaaatta 1320
tcgatgagcg tggtggttat gccgatcgcg tcacactacg tctgaacgtc gaaaacccga 1380
aactgtggag cgccgaaatc ccgaatctct atcgtgcggt ggttgaactg cacaccgccg 1440
acggcacgct gattgaagca gaagcctgcg atgtcggttt ccgcgaggtg cggattgaaa 1500
atggtctgct gctgctgaac ggcaagccgt tgctgattcg aggcgttaac cgtcacgagc 1560
atcatcctct gcatggtcag gtcatggatg agcagacgat ggtgcaggat atcctgctga 1620
tgaagcagaa caactttaac gccgtgcgct gttcgcatta tccgaaccat ccgctgtggt 1680
acacgctgtg cgaccgctac ggcctgtatg tggtggatga agccaatatt gaaacccacg 1740
gcatggtgcc aatgaatcgt ctgaccgatg atccgcgctg gctaccggcg atgagcgaac 1800
gcgtaacgcg aatggtgcag cgcgatcgta atcacccgag tgtgatcatc tggtcgctgg 1860
ggaatgaatc aggccacggc gctaatcacg acgcgctgta tcgctggatc aaatctgtcg 1920
atccttcccg cccggtgcag tatgaaggcg gcggagccga caccacggcc accgatatta 1980
tttgcccgat gtacgcgcgc gtggatgaag accagccctt cccggctgtg ccgaaatggt 2040
ccatcaaaaa atggctttcg ctacctggag agacgcgccc gctgatcctt tgcgaatacg 2100
cccacgcgat gggtaacagt cttggcggtt tcgctaaata ctggcaggcg tttcgtcagt 2160
atccccgttt acagggcggc ttcgtctggg actgggtgga tcagtcgctg attaaatatg 2220
atgaaaacgg caacccgtgg tcggcttacg gcggtgattt tggcgatacg ccgaacgatc 2280
gccagttctg tatgaacggt ctggtctttg ccgaccgcac gccgcatcca gcgctgacgg 2340
aagcaaaaca ccagcagcag tttttccagt tccgtttatc cgggcaaacc atcgaagtga 2400
ccagcgaata cctgttccgt catagcgata acgagctcct gcactggatg gtggcgctgg 2460
atggtaagcc gctggcaagc ggtgaagtgc ctctggatgt cgctccacaa ggtaaacagt 2520
tgattgaact gcctgaacta ccgcagccgg agagcgccgg gcaactctgg ctcacagtac 2580
gcgtagtgca accgaacgcg accgcatggt cagaagccgg gcacatcagc gcctggcagc 2640
agtggcgtct ggcggaaaac ctcagtgtga cgctccccgc cgcgtcccac gccatcccgc 2700
atctgaccac cagcgaaatg gatttttgca tcgagctggg taataagcgt tggcaattta 2760
accgccagtc aggctttctt tcacagatgt ggattggcga taaaaaacaa ctgctgacgc 2820
cgctgcgcga tcagttcacc cgtgcaccgc tggataacga cattggcgta agtgaagcga 2880
cccgcattga ccctaacgcc tgggtcgaac gctggaaggc ggcgggccat taccaggccg 2940
aagcagcgtt gttgcagtgc acggcagata cacttgctga tgcggtgctg attacgaccg 3000
ctcacgcgtg gcagcatcag gggaaaacct tatttatcag ccggaaaacc taccggattg 3060
atggtagtgg tcaaatggcg attaccgttg atgttgaagt ggcgagcgat acaccgcatc 3120
cggcgcggat tggcctgaac tgccagctgg cgcaggtagc agagcgggta aactggctcg 3180
gattagggcc gcaagaaaac tatcccgacc gccttactgc cgcctgtttt gaccgctggg 3240
atctgccatt gtcagacatg tataccccgt acgtcttccc gagcgaaaac ggtctgcgct 3300
gcgggacgcg cgaattgaat tatggcccac accagtggcg cggcgacttc cagttcaaca 3360
tcagccgcta cagtcaacag caactgatgg aaaccagcca tcgccatctg ctgcacgcgg 3420
aagaaggcac atggctgaat atcgacggtt tccatatggg gattggtggc gacgactcct 3480
ggagcccgtc agtatcggcg gaattccagc tgagcgccgg tcgctaccat taccagttgg 3540
tctggtgtca aaaataataa taaccgggca ggggggatct ttgtgaagga accttacttc 3600
tgtggtgtga cataattgga caaactacct acagagattt aaagctctaa ggtaaatata 3660
aaatttttaa gtgtataatg tgttaaacta ctgattctaa ttgtttgtgt attttagatt 3720
ccaacctatg gaactgatga atgggagcag tggtggaatg ccagatccag acatgataag 3780
atacattgat gagtttggac aaaccacaac tagaatgcag tgaaaaaaat gctttatttg 3840
tgaaatttgt gatgctattg ctttatttgt aaccattata agctgcaata aacaagttaa 3900
caacaacaat tgcattcatt ttatgtttca ggttcagggg gaggtgtggg aggtttttta 3960
aagcaagtaa aacctctaca aatgtggtat ggctgattat gatctgcggc cgcagggcct 4020
cgtgatacgc ctatttttat aggttaatgt catgataata atggtttctt agacgtcagg 4080
tggcactttt cggggaaatg tgcgcggaac ccctatttgt ttatttttct aaatacattc 4140
aaatatgtat ccgctcatga gacaataacc ctgataaatg cttcaataat attgaaaaag 4200
gaagagtatg agtattcaac atttccgtgt cgcccttatt cccttttttg cggcattttg 4260
ccttcctgtt tttgctcacc cagaaacgct ggtgaaagta aaagatgctg aagatcagtt 4320
gggtgcacga gtgggttaca tcgaactgga tctcaacagc ggtaagatcc ttgagagttt 4380
tcgccccgaa gaacgttttc caatgatgag cacttttaaa gttctgctat gtggcgcggt 4440
attatcccgt attgacgccg ggcaagagca actcggtcgc cgcatacact attctcagaa 4500
tgacttggtt gagtactcac cagtcacaga aaagcatctt acggatggca tgacagtaag 4560
agaattatgc agtgctgcca taaccatgag tgataacact gcggccaact tacttctgac 4620
aacgatcgga ggaccgaagg agctaaccgc ttttttgcac aacatggggg atcatgtaac 4680
tcgccttgat cgttgggaac cggagctgaa tgaagccata ccaaacgacg agcgtgacac 4740
cacgatgcct gtagcaatgg caacaacgtt gcgcaaacta ttaactggcg aactacttac 4800
tctagcttcc cggcaacaat taatagactg gatggaggcg gataaagttg caggaccact 4860
tctgcgctcg gcccttccgg ctggctggtt tattgctgat aaatctggag ccggtgagcg 4920
tgggtctcgc ggtatcattg cagcactggg gccagatggt aagccctccc gtatcgtagt 4980
tatctacacg acggggagtc aggcaactat ggatgaacga aatagacaga tcgctgagat 5040
aggtgcctca ctgattaagc attggtaact gtcagaccaa gtttactcat atatacttta 5100
gattgattta aaacttcatt tttaatttaa aaggatctag gtgaagatcc tttttgataa 5160
tctcatgacc aaaatccctt aacgtgagtt ttcgttccac tgagcgtcag accccgtaga 5220

aaagatcaaa ggatcttctt gagatccttt ttttctgcgc gtaatctgct gcttgcaaac 5280
aaaaaaacca ccgctaccag cggtggtttg tttgccggat caagagctac caactctttt 5340
tccgaaggta actggcttca gcagagcgca gataccaaat actgtccttc tagtgtagcc 5400
gtagttaggc caccacttca agaactctgt agcaccgcct acatacctcg ctctgctaat 5460
cctgttacca gtggctgctg ccagtggcga taagtcgtgt cttaccgggt tggactcaag 5520
acgatagtta ccggataagg cgcagcggtc gggctgaacg gggggttcgt gcacacagcc 5580
cagcttggag cgaacgacct acaccgaact gagataccta cagcgtgagc tatgagaaag 5640
cgccacgctt cccgaaggga gaaaggcgga caggtatccg gtaagcggca gggtcggaac 5700
aggagagcgc acgagggagc ttccaggggg aaacgcctgg tatctttata gtcctgtcgg 5760
gtttcgccac ctctgacttg agcgtcgatt tttgtgatgc tcgtcagggg ggcggagcct 5820
atggaaaaac gccagcaacg cggccttttt acggttcctg gccttttgct ggccttttgc 5880
tcacatgttc tttcctgcgt tatcccctga ttctgtggat aaccgtatta ccgcctttga 5940
gtgagctgat accgctcgcc gcagccgaac gaccgagcgc agcgagtcag tgagcgagga 6000
agcggaagag cgcccaatac gcaaaccgcc tctccccgcg cgttggccga ttcattaatg 6060
cagctggcac gacaggtttc ccgactggaa agcgggcagt gagcgcaacg caattaatgt 6120
gagttagctc actcattagg caccccaggc tttacacttt atgcttccgg ctcgtatgtt 6180
gtgtggaatt gtgagcggat aacaatttca cacaggaaac agctatgacc atgattacgc 6240
caagctggcg cg 6252

<210> 17
<211> 25
<212> DNA
<213> Artificial Sequence

<220>
<223> Description of Artificial Sequence:
      oligonucleotide P64-1

<400> 17
tcagcaacca ggctccccag caggc 25

<210> 18
<211> 27
<212> DNA
<213> Artificial Sequence

<220>
<223> Description of Artificial Sequence:
      oligonucleotide 64-4

<400> 18
gacgacagta tcggcctcag gaagatc 27

<210> 19
<211> 840
<212> DNA
<213> Artificial Sequence

<220>
<223> Description of Artificial Sequence:
      oligonucleotide 80d

<400> 19
ggtaccgagc tcggatcctc tagtaacggc cgccagtgtg ctggaattcg gcttcagcaa 60
ccaggctccc cagcaggcag aagtatgcaa agcatgcatc tcaattagtc agcaaccagg 120
tgtggaaagt ccccaggctc cccagcaggc agaagtatgc aaagcatgca tctcaattag 180
tcagcaacca tagtcccgcc cctaactccg cccatcccgc ccctaactcc gcccagttcc 240
gcccattctc cgccccatgg ctgactaatt ttttttattt atgcagaggc cgaggccgcc 300
tcggcctagg aacagtcgac gacactgcag agacctactt cactaacaac cggtacagtt 360
cgtggaccag atgggtgagg tggagtacgc gcccggggag cccaaaggtt accccagttg 420
gggcactact cccgaaaacc gcttctggat ccataacttc gtatagcata cattatacga 480
agttataccg ggccaccatg gtcgcgagta gcttggcact ggggttgctt ttgcgnygtc 540
gtgactggga aaaccctggc gttacccaac ttaatcgcct tgcagcacat ccccctttcg 600
ccagctggcg taatagcgaa gaggcccgca ccgatcgccc ttcccaacag ttgcgcagct 660

gaatggcgaa tggcgctttg cctggcttcc ggcaccagaa gcggtgccgg aaagctggct 720
ggagtgcgat cttcctgagg ccgatactgt cgtcaagccg aattctgcag atatccatca 780
cactggcggc cgctcgagca tgcatctaga gggccaattc gccctatagt gagtcgtatt 840

<210> 20
<211> 1842
<212> DNA
<213> Bacteriophage phi-C31

<220>
<221> CDS
<222> (1)..(1839)

<400> 20
atg aca caa ggg gtt gtg acc ggg gtg gac acg tac gcg ggt gct tac 48
Met Thr Gln Gly Val Val Thr Gly Val Asp Thr Tyr Ala Gly Ala Tyr
1               5                   10                  15

gac cgt cag tcg cgc gag cgc gag aat tcg agc gca gca agc cca gcg 96
Asp Arg Gln Ser Arg Glu Arg Glu Asn Ser Ser Ala Ala Ser Pro Ala
            20                  25                  30

aca cag cgt agc gcc aac gaa gac aag gcg gcc gac ctt cag cgc gaa 144
Thr Gln Arg Ser Ala Asn Glu Asp Lys Ala Ala Asp Leu Gln Arg Glu
        35                  40                  45

gtc gag cgc gac ggg ggc cgg ttc agg ttc gtc ggg cat ttc agc gaa 192
Val Glu Arg Asp Gly Gly Arg Phe Arg Phe Val Gly His Phe Ser Glu
    50                  55                  60

gcg ccg ggc acg tcg gcg ttc ggg acg gcg gag cgc ccg gag ttc gaa 240
Ala Pro Gly Thr Ser Ala Phe Gly Thr Ala Glu Arg Pro Glu Phe Glu
65                  70                  75                  80

cgc atc ctg aac gaa tgc cgc gcc ggg cgg ctc aac atg atc att gtc 288
Arg Ile Leu Asn Glu Cys Arg Ala Gly Arg Leu Asn Met Ile Ile Val
                85                  90                  95

tat gac gtg tcg cgc ttc tcg cgc ctg aag gtc atg gac gcg att ccg 336
Tyr Asp Val Ser Arg Phe Ser Arg Leu Lys Val Met Asp Ala Ile Pro
            100                 105                 110

att gtc tcg gaa ttg ctc gcc ctg ggc gtg acg att gtt tcc act cag 384
Ile Val Ser Glu Leu Leu Ala Leu Gly Val Thr Ile Val Ser Thr Gln
        115                 120                 125

gaa ggc gtc ttc cgg cag gga aac gtc atg gac ctg att cac ctg att 432
Glu Gly Val Phe Arg Gln Gly Asn Val Met Asp Leu Ile His Leu Ile
    130                 135                 140

atg cgg ctc gac gcg tcg cac aaa gaa tct tcg ctg aag tcg gcg aag 480
Met Arg Leu Asp Ala Ser His Lys Glu Ser Ser Leu Lys Ser Ala Lys
145                 150                 155                 160

att ctc gac acg aag aac ctt cag cgc gaa ttg ggc ggg tac gtc ggc 528
Ile Leu Asp Thr Lys Asn Leu Gln Arg Glu Leu Gly Gly Tyr Val Gly
                165                 170                 175

ggg aag gcg cct tac ggc ttc gag ctt gtt tcg gag acg aag gag atc 576
Gly Lys Ala Pro Tyr Gly Phe Glu Leu Val Ser Glu Thr Lys Glu Ile
            180                 185                 190

acg cgc aac ggc cga atg gtc aat gtc gtc atc aac aag ctt gcg cac 624
Thr Arg Asn Gly Arg Met Val Asn Val Val Ile Asn Lys Leu Ala His
        195                 200                 205

tcg acc act ccc ctt acc gga ccc ttc gag ttc gag ccc gac gta atc    672
Ser Thr Thr Pro Leu Thr Gly Pro Phe Glu Phe Glu Pro Asp Val Ile
    210                 215                 220

cgg tgg tgg tgg cgt gag atc aag acg cac aaa cac ctt ccc ttc aag    720
Arg Trp Trp Trp Arg Glu Ile Lys Thr His Lys His Leu Pro Phe Lys
225                 230                 235                 240

ccg ggc agt caa gcc gcc att cac ccg ggc agc atc acg ggg ctt tgt    768
Pro Gly Ser Gln Ala Ala Ile His Pro Gly Ser Ile Thr Gly Leu Cys
                245                 250                 255

aag cgc atg gac gct gac gcc gtg ccg acc cgg ggc gag acg att ggg    816
Lys Arg Met Asp Ala Asp Ala Val Pro Thr Arg Gly Glu Thr Ile Gly
            260                 265                 270

aag aag acc gct tca agc gcc tgg gac ccg gca acc gtt atg cga atc    864
Lys Lys Thr Ala Ser Ser Ala Trp Asp Pro Ala Thr Val Met Arg Ile
        275                 280                 285

ctt cgg gac ccg cgt att gcg ggc ttc gcc gct gag gtg atc tac aag    912
Leu Arg Asp Pro Arg Ile Ala Gly Phe Ala Ala Glu Val Ile Tyr Lys
    290                 295                 300

aag aag ccg gac ggc acg ccg acc acg aag att gag ggt tac cgc att    960
Lys Lys Pro Asp Gly Thr Pro Thr Thr Lys Ile Glu Gly Tyr Arg Ile
305                 310                 315                 320

cag cgc gac ccg atc acg ctc cgg ccg gtc gag ctt gat tgc gga ccg    1008
Gln Arg Asp Pro Ile Thr Leu Arg Pro Val Glu Leu Asp Cys Gly Pro
                325                 330                 335

atc atc gag ccc gct gag tgg tat gag ctt cag gcg tgg ttg gac ggc    1056
Ile Ile Glu Pro Ala Glu Trp Tyr Glu Leu Gln Ala Trp Leu Asp Gly
            340                 345                 350

agg ggg cgc ggc aag ggg ctt tcc cgg ggg caa gcc att ctg tcc gcc    1104
Arg Gly Arg Gly Lys Gly Leu Ser Arg Gly Gln Ala Ile Leu Ser Ala
        355                 360                 365

atg gac aag ctg tac tgc gag tgt ggc gcc gtc atg act tcg aag cgc    1152
Met Asp Lys Leu Tyr Cys Glu Cys Gly Ala Val Met Thr Ser Lys Arg
    370                 375                 380

ggg gaa gaa tcg atc aag gac tct tac cgc tgc cgt cgc cgg aag gtg    1200
Gly Glu Glu Ser Ile Lys Asp Ser Tyr Arg Cys Arg Arg Arg Lys Val
385                 390                 395                 400

gtc gac ccg tcc gca cct ggg cag cac gaa ggc acg tgc aac gtc agc    1248
Val Asp Pro Ser Ala Pro Gly Gln His Glu Gly Thr Cys Asn Val Ser
                405                 410                 415

atg gcg gca ctc gac aag ttc gtt gcg gaa cgc atc ttc aac aag atc    1296
Met Ala Ala Leu Asp Lys Phe Val Ala Glu Arg Ile Phe Asn Lys Ile
            420                 425                 430

agg cac gcc gaa ggc gac gaa gag acg ttg gcg ctt ctg tgg gaa gcc    1344
Arg His Ala Glu Gly Asp Glu Glu Thr Leu Ala Leu Leu Trp Glu Ala
        435                 440                 445

gcc cga cgc ttc ggc aag ctc act gag gcg cct gag aag agc ggc gaa    1392
Ala Arg Arg Phe Gly Lys Leu Thr Glu Ala Pro Glu Lys Ser Gly Glu
    450                 455                 460

cgg gcg aac ctt gtt gcg gag cgc gcc gac gcc ctg aac gcc ctt gaa     1440
Arg Ala Asn Leu Val Ala Glu Arg Ala Asp Ala Leu Asn Ala Leu Glu
465                 470                 475                 480

gag ctg tac gaa gac cgc gcg gca ggc gcg tac gac gga ccc gtt ggc     1488
Glu Leu Tyr Glu Asp Arg Ala Ala Gly Ala Tyr Asp Gly Pro Val Gly
                485                 490                 495

agg aag cac ttc cgg aag caa cag gca gcg ctg acg ctc cgg cag caa     1536
Arg Lys His Phe Arg Lys Gln Gln Ala Ala Leu Thr Leu Arg Gln Gln
            500                 505                 510

ggg gcg gaa gag cgg ctt gcc gaa ctt gaa gcc gcc gaa gcc ccg aag     1584
Gly Ala Glu Glu Arg Leu Ala Glu Leu Glu Ala Ala Glu Ala Pro Lys
        515                 520                 525

ctt ccc ctt gac caa tgg ttc ccc gaa gac gcc gac gct gac ccg acc     1632
Leu Pro Leu Asp Gln Trp Phe Pro Glu Asp Ala Asp Ala Asp Pro Thr
    530                 535                 540

ggc cct aag tcg tgg tgg ggg cgc gcg tca gta gac gac aag cgc gtg     1680
Gly Pro Lys Ser Trp Trp Gly Arg Ala Ser Val Asp Asp Lys Arg Val
545                 550                 555                 560

ttc gtc ggg ctc ttc gta gac aag atc gtt gtc acg aag tcg act acg     1728
Phe Val Gly Leu Phe Val Asp Lys Ile Val Val Thr Lys Ser Thr Thr
                565                 570                 575

ggc agg ggg cag gga acg ccc atc gag aag cgc gct tcg atc acg tgg     1776
Gly Arg Gly Gln Gly Thr Pro Ile Glu Lys Arg Ala Ser Ile Thr Trp
            580                 585                 590

gcg aag ccg ccg acc gac gac gac gaa gac gac gcc cag gac ggc acg     1824
Ala Lys Pro Pro Thr Asp Asp Asp Glu Asp Asp Ala Gln Asp Gly Thr
        595                 600                 605

gaa gac gta gcg gcg tag                                             1842
Glu Asp Val Ala Ala
    610


<210> 21
<211> 613
<212> PRT
<213> Bacteriophage phi-C31

<400> 21
Met Thr Gln Gly Val Val Thr Gly Val Asp Thr Tyr Ala Gly Ala Tyr
1               5                   10                  15

Asp Arg Gln Ser Arg Glu Arg Glu Asn Ser Ser Ala Ala Ser Pro Ala
            20                  25                  30

Thr Gln Arg Ser Ala Asn Glu Asp Lys Ala Ala Asp Leu Gln Arg Glu
        35                  40                  45

Val Glu Arg Asp Gly Gly Arg Phe Arg Phe Val Gly His Phe Ser Glu
    50                  55                  60

Ala Pro Gly Thr Ser Ala Phe Gly Thr Ala Glu Arg Pro Glu Phe Glu
65                  70                  75                  80

Arg Ile Leu Asn Glu Cys Arg Ala Gly Arg Leu Asn Met Ile Ile Val
                85                  90                  95

Tyr Asp Val Ser Arg Phe Ser Arg Leu Lys Val Met Asp Ala Ile Pro
            100                 105                 110

Ile Val Ser Glu Leu Leu Ala Leu Gly Val Thr Ile Val Ser Thr Gln
        115                 120                 125

Glu Gly Val Phe Arg Gln Gly Asn Val Met Asp Leu Ile His Leu Ile
    130                 135                 140

Met Arg Leu Asp Ala Ser His Lys Glu Ser Ser Leu Lys Ser Ala Lys
145                 150                 155                 160

Ile Leu Asp Thr Lys Asn Leu Gln Arg Glu Leu Gly Gly Tyr Val Gly
                165                 170                 175

Gly Lys Ala Pro Tyr Gly Phe Glu Leu Val Ser Glu Thr Lys Glu Ile
            180                 185                 190

Thr Arg Asn Gly Arg Met Val Asn Val Val Ile Asn Lys Leu Ala His
        195                 200                 205

Ser Thr Thr Pro Leu Thr Gly Pro Phe Glu Phe Glu Pro Asp Val Ile
    210                 215                 220

Arg Trp Trp Trp Arg Glu Ile Lys Thr His Lys His Leu Pro Phe Lys
225                 230                 235                 240

Pro Gly Ser Gln Ala Ala Ile His Pro Gly Ser Ile Thr Gly Leu Cys
                245                 250                 255

Lys Arg Met Asp Ala Asp Ala Val Pro Thr Arg Gly Glu Thr Ile Gly
            260                 265                 270

Lys Lys Thr Ala Ser Ser Ala Trp Asp Pro Ala Thr Val Met Arg Ile
        275                 280                 285

Leu Arg Asp Pro Arg Ile Ala Gly Phe Ala Ala Glu Val Ile Tyr Lys
    290                 295                 300

Lys Lys Pro Asp Gly Thr Pro Thr Thr Lys Ile Glu Gly Tyr Arg Ile
305                 310                 315                 320

Gln Arg Asp Pro Ile Thr Leu Arg Pro Val Glu Leu Asp Cys Gly Pro
                325                 330                 335

Ile Ile Glu Pro Ala Glu Trp Tyr Glu Leu Gln Ala Trp Leu Asp Gly
            340                 345                 350

Arg Gly Arg Gly Lys Gly Leu Ser Arg Gly Gln Ala Ile Leu Ser Ala
        355                 360                 365

Met Asp Lys Leu Tyr Cys Glu Cys Gly Ala Val Met Thr Ser Lys Arg
    370                 375                 380

Gly Glu Glu Ser Ile Lys Asp Ser Tyr Arg Cys Arg Arg Arg Lys Val
385                 390                 395                 400

Val Asp Pro Ser Ala Pro Gly Gln His Glu Gly Thr Cys Asn Val Ser
                405                 410                 415

Met Ala Ala Leu Asp Lys Phe Val Ala Glu Arg Ile Phe Asn Lys Ile
            420                 425                 430

Arg His Ala Glu Gly Asp Glu Glu Thr Leu Ala Leu Leu Trp Glu Ala
        435                 440                 445

Ala Arg Arg Phe Gly Lys Leu Thr Glu Ala Pro Glu Lys Ser Gly Glu
    450                 455                 460

Arg Ala Asn Leu Val Ala Glu Arg Ala Asp Ala Leu Asn Ala Leu Glu
465                 470                 475                 480

Glu Leu Tyr Glu Asp Arg Ala Ala Gly Ala Tyr Asp Gly Pro Val Gly
                485                 490                 495

Arg Lys His Phe Arg Lys Gln Gln Ala Ala Leu Thr Leu Arg Gln Gln
            500                 505                 510

Gly Ala Glu Glu Arg Leu Ala Glu Leu Glu Ala Ala Glu Ala Pro Lys
        515                 520                 525

Leu Pro Leu Asp Gln Trp Phe Pro Glu Asp Ala Asp Ala Asp Pro Thr
    530                 535                 540

Gly Pro Lys Ser Trp Trp Gly Arg Ala Ser Val Asp Asp Lys Arg Val
545                 550                 555                 560

Phe Val Gly Leu Phe Val Asp Lys Ile Val Val Thr Lys Ser Thr Thr
                565                 570                 575

Gly Arg Gly Gln Gly Thr Pro Ile Glu Lys Arg Ala Ser Ile Thr Trp
            580                 585                 590

Ala Lys Pro Pro Thr Asp Asp Asp Glu Asp Asp Ala Gln Asp Gly Thr
        595                 600                 605

Glu Asp Val Ala Ala
    610


<210> 22
<211> 1863
<212> DNA
<213> Artificial Sequence

<220>
<223> Description of Artificial Sequence: DNA sequence
      coding for fusion protein C31-Int(NLS)

<220>
<221> CDS
<222> (1)..(1860)

<400> 22
atg aca caa ggg gtt gtg acc ggg gtg gac acg tac gcg ggt gct tac      48
Met Thr Gln Gly Val Val Thr Gly Val Asp Thr Tyr Ala Gly Ala Tyr
  1               5                  10                  15

gac cgt cag tcg cgc gag cgc gag aat tcg agc gca gca agc cca gcg      96
Asp Arg Gln Ser Arg Glu Arg Glu Asn Ser Ser Ala Ala Ser Pro Ala
             20                  25                  30

aca cag cgt agc gcc aac gaa gac aag gcg gcc gac ctt cag cgc gaa     144
Thr Gln Arg Ser Ala Asn Glu Asp Lys Ala Ala Asp Leu Gln Arg Glu
         35                  40                  45

gtc gag cgc gac ggg ggc cgg ttc agg ttc gtc ggg cat ttc agc gaa     192
Val Glu Arg Asp Gly Gly Arg Phe Arg Phe Val Gly His Phe Ser Glu
     50                  55                  60

gcg ccg ggc acg tcg gcg ttc ggg acg gcg gag cgc ccg gag ttc gaa     240
Ala Pro Gly Thr Ser Ala Phe Gly Thr Ala Glu Arg Pro Glu Phe Glu
 65                  70                  75                  80

cgc atc ctg aac gaa tgc cgc gcc ggg cgg ctc aac atg atc att gtc     288
Arg Ile Leu Asn Glu Cys Arg Ala Gly Arg Leu Asn Met Ile Ile Val

                85                  90                  95

tat gac gtg tcg cgc ttc tcg cgc ctg aag gtc atg gac gcg att ccg 336
Tyr Asp Val Ser Arg Phe Ser Arg Leu Lys Val Met Asp Ala Ile Pro
            100                 105                 110

att gtc tcg gaa ttg ctc gcc ctg ggc gtg acg att gtt tcc act cag 384
Ile Val Ser Glu Leu Leu Ala Leu Gly Val Thr Ile Val Ser Thr Gln
        115                 120                 125

gaa ggc gtc ttc cgg cag gga aac gtc atg gac ctg att cac ctg att 432
Glu Gly Val Phe Arg Gln Gly Asn Val Met Asp Leu Ile His Leu Ile

    130                 135                 140

atg cgg ctc gac gcg tcg cac aaa gaa tct tcg ctg aag tcg gcg aag 480
Met Arg Leu Asp Ala Ser His Lys Glu Ser Ser Leu Lys Ser Ala Lys
145                 150                 155                 160

att ctc gac acg aag aac ctt cag cgc gaa ttg ggc ggg tac gtc ggc 528
Ile Leu Asp Thr Lys Asn Leu Gln Arg Glu Leu Gly Gly Tyr Val Gly
                165                 170                 175

ggg aag gcg cct tac ggc ttc gag ctt gtt tcg gag acg aag gag atc 576
Gly Lys Ala Pro Tyr Gly Phe Glu Leu Val Ser Glu Thr Lys Glu Ile
            180                 185                 190

acg cgc aac ggc cga atg gtc aat gtc gtc atc aac aag ctt gcg cac 624
Thr Arg Asn Gly Arg Met Val Asn Val Val Ile Asn Lys Leu Ala His
        195                 200                 205

tcg acc act ccc ctt acc gga ccc ttc gag ttc gag ccc gac gta atc 672
Ser Thr Thr Pro Leu Thr Gly Pro Phe Glu Phe Glu Pro Asp Val Ile
    210                 215                 220

cgg tgg tgg tgg cgt gag atc aag acg cac aaa cac ctt ccc ttc aag 720
Arg Trp Trp Trp Arg Glu Ile Lys Thr His Lys His Leu Pro Phe Lys
225                 230                 235                 240

ccg ggc agt caa gcc gcc att cac ccg ggc agc atc acg ggg ctt tgt 768
Pro Gly Ser Gln Ala Ala Ile His Pro Gly Ser Ile Thr Gly Leu Cys
                245                 250                 255

aag cgc atg gac gct gac gcc gtg ccg acc cgg ggc gag acg att ggg 816
Lys Arg Met Asp Ala Asp Ala Val Pro Thr Arg Gly Glu Thr Ile Gly
            260                 265                 270

aag aag acc gct tca agc gcc tgg gac ccg gca acc gtt atg cga atc 864
Lys Lys Thr Ala Ser Ser Ala Trp Asp Pro Ala Thr Val Met Arg Ile
        275                 280                 285

ctt cgg gac ccg cgt att gcg ggc ttc gcc gct gag gtg atc tac aag 912
Leu Arg Asp Pro Arg Ile Ala Gly Phe Ala Ala Glu Val Ile Tyr Lys
    290                 295                 300

aag aag ccg gac ggc acg ccg acc acg aag att gag ggt tac cgc att 960
Lys Lys Pro Asp Gly Thr Pro Thr Thr Lys Ile Glu Gly Tyr Arg Ile
305                 310                 315                 320

cag cgc gac ccg atc acg ctc cgg ccg gtc gag ctt gat tgc gga ccg 1008
Gln Arg Asp Pro Ile Thr Leu Arg Pro Val Glu Leu Asp Cys Gly Pro
                325                 330                 335

atc atc gag ccc gct gag tgg tat gag ctt cag gcg tgg ttg gac ggc 1056
Ile Ile Glu Pro Ala Glu Trp Tyr Glu Leu Gln Ala Trp Leu Asp Gly
            340                 345                 350

agg ggg cgc ggc aag ggg ctt tcc cgg ggg caa gcc att ctg tcc gcc 1104
Arg Gly Arg Gly Lys Gly Leu Ser Arg Gly Gln Ala Ile Leu Ser Ala
        355                 360                 365

atg gac aag ctg tac tgc gag tgt ggc gcc gtc atg act tcg aag cgc 1152
Met Asp Lys Leu Tyr Cys Glu Cys Gly Ala Val Met Thr Ser Lys Arg
    370                 375                 380

ggg gaa gaa tcg atc aag gac tct tac cgc tgc cgt cgc cgg aag gtg 1200
Gly Glu Glu Ser Ile Lys Asp Ser Tyr Arg Cys Arg Arg Arg Lys Val
385                 390                 395                 400

gtc gac ccg tcc gca cct ggg cag cac gaa ggc acg tgc aac gtc agc 1248
Val Asp Pro Ser Ala Pro Gly Gln His Glu Gly Thr Cys Asn Val Ser
                405                 410                 415

atg gcg gca ctc gac aag ttc gtt gcg gaa cgc atc ttc aac aag atc 1296
Met Ala Ala Leu Asp Lys Phe Val Ala Glu Arg Ile Phe Asn Lys Ile
            420                 425                 430

agg cac gcc gaa ggc gac gaa gag acg ttg gcg ctt ctg tgg gaa gcc 1344
Arg His Ala Glu Gly Asp Glu Glu Thr Leu Ala Leu Leu Trp Glu Ala
        435                 440                 445

gcc cga cgc ttc ggc aag ctc act gag gcg cct gag aag agc ggc gaa 1392
Ala Arg Arg Phe Gly Lys Leu Thr Glu Ala Pro Glu Lys Ser Gly Glu
    450                 455                 460

cgg gcg aac ctt gtt gcg gag cgc gcc gac gcc ctg aac gcc ctt gaa 1440
Arg Ala Asn Leu Val Ala Glu Arg Ala Asp Ala Leu Asn Ala Leu Glu
465                 470                 475                 480

gag ctg tac gaa gac cgc gcg gca ggc gcg tac gac gga ccc gtt ggc 1488
Glu Leu Tyr Glu Asp Arg Ala Ala Gly Ala Tyr Asp Gly Pro Val Gly
                485                 490                 495

agg aag cac ttc cgg aag caa cag gca gcg ctg acg ctc cgg cag caa 1536
Arg Lys His Phe Arg Lys Gln Gln Ala Ala Leu Thr Leu Arg Gln Gln
            500                 505                 510

ggg gcg gaa gag cgg ctt gcc gaa ctt gaa gcc gcc gaa gcc ccg aag 1584
Gly Ala Glu Glu Arg Leu Ala Glu Leu Glu Ala Ala Glu Ala Pro Lys
        515                 520                 525

ctt ccc ctt gac caa tgg ttc ccc gaa gac gcc gac gct gac ccg acc 1632
Leu Pro Leu Asp Gln Trp Phe Pro Glu Asp Ala Asp Ala Asp Pro Thr
    530                 535                 540

ggc cct aag tcg tgg tgg ggg cgc gcg tca gta gac gac aag cgc gtg 1680
Gly Pro Lys Ser Trp Trp Gly Arg Ala Ser Val Asp Asp Lys Arg Val
545                 550                 555                 560

ttc gtc ggg ctc ttc gta gac aag atc gtt gtc acg aag tcg act acg 1728
Phe Val Gly Leu Phe Val Asp Lys Ile Val Val Thr Lys Ser Thr Thr
                565                 570                 575

ggc agg ggg cag gga acg ccc atc gag aag cgc gct tcg atc acg tgg 1776
Gly Arg Gly Gln Gly Thr Pro Ile Glu Lys Arg Ala Ser Ile Thr Trp
            580                 585                 590

gcg aag ccg ccg acc gac gac gac gaa gac gac gcc cag gac ggc acg 1824
Ala Lys Pro Pro Thr Asp Asp Asp Glu Asp Asp Ala Gln Asp Gly Thr
        595                 600                 605

gaa gac gta gcg gcg cct aag aag aag agg aag gtt tag 1863
Glu Asp Val Ala Ala Pro Lys Lys Lys Arg Lys Val

610                    615                    620

<210> 23
<211> 620
<212> PRT
<213> Artificial Sequence
<223> Description of Artificial Sequence: DNA sequence
      coding for fusion protein C31-Int(NLS)

<400> 23
Met Thr Gln Gly Val Val Thr Gly Val Asp Thr Tyr Ala Gly Ala Tyr
1               5                   10                  15

Asp Arg Gln Ser Arg Glu Arg Glu Asn Ser Ser Ala Ala Ser Pro Ala
            20                  25                  30

Thr Gln Arg Ser Ala Asn Glu Asp Lys Ala Ala Asp Leu Gln Arg Glu
        35                  40                  45

Val Glu Arg Asp Gly Gly Arg Phe Arg Phe Val Gly His Phe Ser Glu
    50                  55                  60

Ala Pro Gly Thr Ser Ala Phe Gly Thr Ala Glu Arg Pro Glu Phe Glu
65                  70                  75                  80

Arg Ile Leu Asn Glu Cys Arg Ala Gly Arg Leu Asn Met Ile Ile Val
                85                  90                  95

Tyr Asp Val Ser Arg Phe Ser Arg Leu Lys Val Met Asp Ala Ile Pro
            100                 105                 110

Ile Val Ser Glu Leu Leu Ala Leu Gly Val Thr Ile Val Ser Thr Gln
        115                 120                 125

Glu Gly Val Phe Arg Gln Gly Asn Val Met Asp Leu Ile His Leu Ile
    130                 135                 140

Met Arg Leu Asp Ala Ser His Lys Glu Ser Ser Leu Lys Ser Ala Lys
145                 150                 155                 160

Ile Leu Asp Thr Lys Asn Leu Gln Arg Glu Leu Gly Gly Tyr Val Gly
                165                 170                 175

Gly Lys Ala Pro Tyr Gly Phe Glu Leu Val Ser Glu Thr Lys Glu Ile
            180                 185                 190

Thr Arg Asn Gly Arg Met Val Asn Val Val Ile Asn Lys Leu Ala His
        195                 200                 205

Ser Thr Thr Pro Leu Thr Gly Pro Phe Glu Phe Glu Pro Asp Val Ile
    210                 215                 220

Arg Trp Trp Trp Arg Glu Ile Lys Thr His Lys His Leu Pro Phe Lys
225                 230                 235                 240

Pro Gly Ser Gln Ala Ala Ile His Pro Gly Ser Ile Thr Gly Leu Cys
                245                 250                 255

Lys Arg Met Asp Ala Asp Ala Val Pro Thr Arg Gly Glu Thr Ile Gly
            260                 265                 270

Lys Lys Thr Ala Ser Ser Ala Trp Asp Pro Ala Thr Val Met Arg Ile
        275                 280                 285

Leu Arg Asp Pro Arg Ile Ala Gly Phe Ala Ala Glu Val Ile Tyr Lys
    290                 295                 300

Lys Lys Pro Asp Gly Thr Pro Thr Thr Lys Ile Glu Gly Tyr Arg Ile
305                 310                 315                 320

Gln Arg Asp Pro Ile Thr Leu Arg Pro Val Glu Leu Asp Cys Gly Pro
                325                 330                 335

Ile Ile Glu Pro Ala Glu Trp Tyr Glu Leu Gln Ala Trp Leu Asp Gly
            340                 345                 350

Arg Gly Arg Gly Lys Gly Leu Ser Arg Gly Gln Ala Ile Leu Ser Ala
        355                 360                 365

Met Asp Lys Leu Tyr Cys Glu Cys Gly Ala Val Met Thr Ser Lys Arg
    370                 375                 380

Gly Glu Glu Ser Ile Lys Asp Ser Tyr Arg Cys Arg Arg Arg Lys Val
385                 390                 395                 400

Val Asp Pro Ser Ala Pro Gly Gln His Glu Gly Thr Cys Asn Val Ser
                405                 410                 415

Met Ala Ala Leu Asp Lys Phe Val Ala Glu Arg Ile Phe Asn Lys Ile
            420                 425                 430

Arg His Ala Glu Gly Asp Glu Glu Thr Leu Ala Leu Leu Trp Glu Ala
        435                 440                 445

Ala Arg Arg Phe Gly Lys Leu Thr Glu Ala Pro Glu Lys Ser Gly Glu
    450                 455                 460

Arg Ala Asn Leu Val Ala Glu Arg Ala Asp Ala Leu Asn Ala Leu Glu
465                 470                 475                 480

Glu Leu Tyr Glu Asp Arg Ala Ala Gly Ala Tyr Asp Gly Pro Val Gly
                485                 490                 495

Arg Lys His Phe Arg Lys Gln Gln Ala Ala Leu Thr Leu Arg Gln Gln
            500                 505                 510

Gly Ala Glu Glu Arg Leu Ala Glu Leu Glu Ala Ala Glu Ala Pro Lys
        515                 520                 525

Leu Pro Leu Asp Gln Trp Phe Pro Glu Asp Ala Asp Ala Asp Pro Thr
    530                 535                 540

Gly Pro Lys Ser Trp Trp Gly Arg Ala Ser Val Asp Asp Lys Arg Val
545                 550                 555                 560

Phe Val Gly Leu Phe Val Asp Lys Ile Val Val Thr Lys Ser Thr Thr
                565                 570                 575

Gly Arg Gly Gln Gly Thr Pro Ile Glu Lys Arg Ala Ser Ile Thr Trp
            580                 585                 590

Ala Lys Pro Pro Thr Asp Asp Asp Glu Asp Asp Ala Gln Asp Gly Thr
        595                 600                 605

Glu Asp Val Ala Ala Pro Lys Lys Lys Arg Lys Val
    610                 615                 620

<210> 24
<211> 43
<212> PRT
<213> Artificial Sequence

<220>
<223> Description of Artificial Sequence: NLS

<400> 24
Met Lys Lys Lys Lys Lys Lys Lys Lys Lys Lys Lys Cys Arg Leu Lys
1               5                   10                  15

Lys Leu Lys Cys Ser Lys Glu Lys Pro Lys Cys Ala Lys Cys Leu Lys
            20                  25                  30

Lys Lys Lys Lys Arg Arg Arg Lys Thr Lys Arg
        35                  40

<210> 25
<211> 10
<212> PRT
<213> Artificial Sequence

<220>
<223> Description of Artificial Sequence: NLS

<400> 25
Ile Lys Tyr Phe Lys Lys Phe Pro Lys Asp
1               5                   10

<210> 26
<211> 14
<212> PRT
<213> Artificial Sequence

<220>
<223> Description of Artificial Sequence: NLS

<400> 26
Met Thr Gly Ser Lys Thr Arg Lys His Arg Gly Ser Gly Ala
1               5                   10

<210> 27
<211> 14
<212> PRT
<213> Artificial Sequence

<220>
<223> Description of Artificial Sequence: NLS

<400> 27
Met Thr Gly Ser Lys His Arg Lys His Pro Gly Ser Gly Ala
1               5                   10

<210> 28
<211> 7
<212> PRT
<213> Artificial Sequence

<220>
<223> Description of Artificial Sequence: NLS

<400> 28
Gly Lys Lys Arg Ser Lys Ala
1               5

<210> 29

<211> 14
<212> PRT
<213> Artificial Sequence

<220>
<223> Description of Artificial Sequence: NLS

<400> 29
Pro Lys Lys Ala Arg Glu Asp Val Ser Arg Lys Arg Pro Arg
  1               5                   10


<210> 30
<211> 11
<212> PRT
<213> Artificial Sequence

<220>
<223> Description of Artificial Sequence: NLS

<400> 30
Ala Pro Lys Arg Lys Ser Gly Val Ser Lys Cys
  1               5                   10


<210> 31
<211> 12
<212> PRT
<213> Artificial Sequence

<220>
<223> Description of Artificial Sequence: NLS

<400> 31
Glu Glu Asp Gly Pro Gln Lys Lys Lys Arg Arg Leu
  1               5                   10


<210> 32
<211> 8
<212> PRT
<213> Artificial Sequence

<220>
<223> Description of Artificial Sequence: NLS

<400> 32
Ala Pro Thr Lys Arg Lys Gly Ser
  1               5


<210> 33
<211> 7
<212> PRT
<213> Artificial Sequence

<220>
<223> Description of Artificial Sequence: NLS

<400> 33
Pro Asn Lys Lys Lys Arg Lys
  1               5


<210> 34

<211> 5
<212> PRT
<213> Artificial Sequence

<220>
<223> Description of Artificial Sequence: NLS

<400> 34
Lys Arg Pro Arg Pro
 1               5


<210> 35
<211> 11
<212> PRT
<213> Artificial Sequence

<220>
<223> Description of Artificial Sequence: NLS

<400> 35
Cys Gly Gly Leu Ser Ser Lys Arg Pro Arg Pro
 1               5                   10


<210> 36
<211> 19
<212> PRT
<213> Artificial Sequence

<220>
<223> Description of Artificial Sequence: NLS

<400> 36
Pro Pro Lys Lys Arg Met Arg Arg Arg Ile Glu Pro Lys Lys Lys Lys
 1               5                   10                  15

Lys Arg Pro


<210> 37
<211> 11
<212> PRT
<213> Artificial Sequence

<220>
<223> Description of Artificial Sequence: NLS

<400> 37
Pro Phe Leu Asp Arg Leu Arg Arg Asp Gln Lys
 1               5                   10


<210> 38
<211> 9
<212> PRT
<213> Artificial Sequence

<220>
<223> Description of Artificial Sequence: NLS

<400> 38
Pro Lys Gln Lys Arg Lys Met Ala Arg
 1               5

<210> 39
<211> 9
<212> PRT
<213> Artificial Sequence

<220>
<223> Description of Artificial Sequence: NLS

<400> 39
Ser Val Thr Lys Lys Arg Lys Leu Glu
  1               5


<210> 40
<211> 11
<212> PRT
<213> Artificial Sequence

<220>
<223> Description of Artificial Sequence: NLS

<400> 40
Cys Gly Gly Ala Ala Lys Arg Val Lys Leu Asp
  1               5                  10


<210> 41
<211> 9
<212> PRT
<213> Artificial Sequence

<220>
<223> Description of Artificial Sequence: NLS

<400> 41
Pro Ala Ala Lys Arg Val Lys Leu Asp
  1               5


<210> 42
<211> 11
<212> PRT
<213> Artificial Sequence

<220>
<223> Description of Artificial Sequence: NLS

<400> 42
Arg Gln Arg Arg Asn Glu Leu Lys Arg Ser Pro
  1               5                  10


<210> 43
<211> 8
<212> PRT
<213> Artificial Sequence

<220>
<223> Description of Artificial Sequence: NLS

<400> 43
Pro Gln Ser Arg Lys Lys Leu Arg
  1               5


<210> 44
<211> 8

<212> PRT
<213> Artificial Sequence

<220>
<223> Description of Artificial Sequence: NLS

<400> 44
Pro Leu Leu Lys Lys Ile Lys Gln
1               5


<210> 45
<211> 7
<212> PRT
<213> Artificial Sequence

<220>
<223> Description of Artificial Sequence: NLS

<400> 45
Pro Gln Pro Lys Lys Lys Pro
1               5


<210> 46
<211> 9
<212> PRT
<213> Artificial Sequence

<220>
<223> Description of Artificial Sequence: NLS

<400> 46
Ser Lys Arg Val Ala Lys Arg Lys Leu
1               5


<210> 47
<211> 9
<212> PRT
<213> Artificial Sequence

<220>
<223> Description of Artificial Sequence: NLS

<400> 47
Ala Ser Lys Ser Arg Lys Arg Lys Leu
1               5


<210> 48
<211> 16
<212> PRT
<213> Artificial Sequence

<220>
<223> Description of Artificial Sequence: NLS

<400> 48
Gly Gly Leu Cys Ser Ala Arg Leu His Arg His Ala Leu Leu Ala Thr
1               5                   10                  15


<210> 49
<211> 8
<212> PRT
<213> Artificial Sequence

<220>
<223> Description of Artificial Sequence: NLS

<400> 49
Arg Lys Thr Lys Lys Lys Ile Lys
1               5


<210> 50
<211> 8
<212> PRT
<213> Artificial Sequence

<220>
<223> Description of Artificial Sequence: NLS

<400> 50
Arg Lys Leu Lys Lys Leu Gly Asn
1               5


<210> 51
<211> 8
<212> PRT
<213> Artificial Sequence

<220>
<223> Description of Artificial Sequence: NLS

<400> 51
Arg Lys Asp Arg Arg Gly Gly Arg
1               5


<210> 52
<211> 18
<212> PRT
<213> Artificial Sequence

<220>
<223> Description of Artificial Sequence: NLS

<400> 52
Asp Thr Arg Glu Lys Lys Lys Phe Leu Lys Arg Arg Leu Leu Arg Leu
1               5                   10                  15

Asp Glu


<210> 53
<211> 7
<212> PRT
<213> Artificial Sequence

<220>
<223> Description of Artificial Sequence: NLS

<400> 53
Pro Lys Lys Lys Arg Lys Val
1               5